(19) Europäisches Patentamt
European Patent Office
Office européen des brevets

(11) **EP 3 842 386 A1**

(12) **EUROPEAN PATENT APPLICATION**
published in accordance with Art. 153(4) EPC

(43) Date of publication:
**30.06.2021 Bulletin 2021/26**

(21) Application number: **19852578.4**

(22) Date of filing: **21.08.2019**

(51) Int Cl.:
*C01B 33/36* (2006.01)    *A61K 9/14* (2006.01)
*A61K 9/20* (2006.01)    *A61K 9/26* (2006.01)
*A61K 9/28* (2006.01)    *A61K 31/14* (2006.01)
*A61K 33/00* (2006.01)    *A61K 33/30* (2006.01)
*A61K 33/34* (2006.01)    *A61K 33/38* (2006.01)
*A61K 45/00* (2006.01)    *A61K 47/02* (2006.01)
*A61K 47/04* (2006.01)    *A61K 47/20* (2006.01)
*A61K 47/22* (2006.01)    *A61P 31/00* (2006.01)
*A61P 31/04* (2006.01)    *C09C 1/00* (2006.01)
*C09C 3/06* (2006.01)    *C09C 3/08* (2006.01)

(86) International application number:
**PCT/JP2019/032552**

(87) International publication number:
**WO 2020/040170 (27.02.2020 Gazette 2020/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
**KH MA MD TN**

(30) Priority: **22.08.2018 JP 2018155738**

(71) Applicant: **Topy Kogyo Kabushiki Kaisha
Tokyo 141-8634 (JP)**

(72) Inventors:
• **SEIKE Ryuichi
  Toyohashi-shi, Aichi 441-8510 (JP)**
• **SUEYOSHI Mai
  Toyohashi-shi, Aichi 441-8510 (JP)**
• **HAYASHI Takayoshi
  Toyohashi-shi, Aichi 441-8510 (JP)**

(74) Representative: **Lippert Stachow Patentanwälte
Rechtsanwälte
Partnerschaft mbB
Frankenforster Strasse 135-137
51427 Bergisch Gladbach (DE)**

(54) **SILICATE COATED BODY**

(57)    A silicate-coated body contains: a mica particle; a first silicate coating at least part of the mica particle; and a functional substance that is carried by the first silicate.

[FIG. 5]

EP 3 842 386 A1

**Description**

Related Application

The present application is based upon and claims the benefit of priority from Japanese Patent Application No. 2018-155738 filed on August 22, 2018, the entire contents of which are incorporated herein by reference.

Technical Field

The present disclosure relates to a silicate-coated body. Particularly, the present disclosure relates to silicate-coated powder (particles).

Background Art

Smectite-group silicates are chemically and thermally stable, and are therefore used for various applications, such as cosmetics, paint, or the like.

Patent Literature 1 discloses a method for synthesizing smectite clay minerals. Unfortunately, the smectite-group silicates produced by the method disclosed in Patent Literature 1 have fine, layered shapes, which poses a problem in handleability. Studies are therefore underway to develop techniques for coating spherical silica particles with smectite to improve handleability (e.g., Patent Literature 2 and Non-Patent Literature 1).

Patent Literature 3 discloses an antibacterial silicate wherein at least a portion of an ion-exchangeable metal contained in a phyllosilicate, such as a smectite or synthetic mica, is substituted by a coordination compound between an organic ligand and at least one type of metal ion selected from the group consisting of silver, copper and zinc.

Citation List

Patent Literature

Patent Literature 1: Japanese Unexamined Patent Publication H07-505112A
Patent Literature 2: Japanese Unexamined Patent Publication 2014-24711A
Patent Literature 3: Japanese Unexamined Patent Publication H03-193707A

Non-Patent Literature 1: Tomohiko Okada et al., "Swellable Microsphere of a Layered Silicate Produced by Using Monodispersed Silica Particles," J. Phys. Chem. C, 2012, vol. 116, pp. 21864-21869.

Summary of Invention

Technical Problem

Silicates, such as mica-group and smectite-group silicates, have high chemical stability, which in turn makes it difficult to chemically modify such silicates and/or modify other substances with such silicates. There is no known method for, for example, modifying another highly chemically stable silicate (e.g., mica) with a smectite-group silicate at the particle level.

As regards the smectite-coated silica particles synthesized according to the method of Patent Literature 2 or Non-Patent Literature 1, the geometry of smectite on the particles is dependent on the shape and size of the spherical silica particles which serve as the substrate. Thus, there are limited applications for such smectite-coated silica particles.

Smectite-group silicates, such as those disclosed in Patent Literature 3, are likely to aggregate when used alone. Aggregation not only makes carrying of antibacterial substances uneven, but also impairs texture or feel to the touch and also impairs handleability. On the other hand, mica-group silicates have poor ion exchangeability; thus, it is difficult to make a mica-group silicate carry an ionic antibacterial substance by ion exchange as disclosed in Patent Literature 3. Therefore, antibacterial silicates as disclosed in Patent Literature 3 cannot offer excellent antibacterial effects, even if ionic antibacterial substances were carried.

Thus, there are demands for a silicate-coated body that is less prone to aggregation and has functionality, while exhibiting characteristics of silicates.

Solution to Problem

According to a first aspect of the present disclosure, a silicate-coated body is provided, the silicate-coated body comprising a mica particle, a first silicate coating at least part of the mica particle, and a functional substance carried by the first silicate.

According to a second aspect of the present disclosure, a silicate-coated body is provided, the silicate-coated body comprising a substrate, silica and/or a silica modified product present on the substrate, a first silicate coating at least part of the substrate via the silica and/or silica modified product, and a functional substance carried by the first silicate.

Advantageous Effects of Invention

According to the present disclosure, it is possible to provide a silicate-coated body that is suppressed from aggregating and is provided with functionality, while exhibiting characteristics of silicates.

Brief Description of Drawings

FIG. 1 is a conceptual diagram of a silicate-coated body according to first and second embodiments of the present disclosure.

FIG. 2 is a conceptual diagram of a silicate-coated body according to first and second embodiments of the present disclosure.

FIG. 3 is a schematic sectional view of a silicate-coated body according to a second embodiment of the present disclosure.

FIG. 4 is a schematic sectional view of a silicate-coated body according to a second embodiment of the present disclosure.

FIG. 5 is a schematic diagram for illustrating a structure of a silicate-coated body according to a second embodiment of the present disclosure, and a mechanism for producing the same.

FIG. 6 is a flowchart of a silicate-coated-body manufacturing method according to a third embodiment.

FIG. 7 is a schematic sectional view of a silicate-coated body according to a fifth embodiment of the present disclosure.

FIG. 8 is a schematic sectional view of a silicate-coated body according to a fifth embodiment of the present disclosure.

FIG. 9 is a flowchart of a silicate-coated-body manufacturing method according to a sixth embodiment.

FIG. 10 is a schematic sectional view of a silicate-coated body according to an eighth embodiment of the present disclosure.

FIG. 11 is a schematic sectional view of a silicate-coated body according to an eighth embodiment of the present disclosure.

FIG. 12 is a flowchart of a silicate-coated-body manufacturing method according to a tenth embodiment.

FIG. 13 illustrates X-ray diffraction patterns of hectorite-coated mica according to Test Examples 1 to 6.

FIG. 14 illustrates an X-ray diffraction pattern of hectorite-coated mica according to Test Example 1.

FIG. 15 illustrates X-ray diffraction patterns of hectorite-coated mica according to Test Example 1 and hectorite within the range $2\theta = 2°$ to $12°$.

FIG. 16 illustrates X-ray diffraction patterns of hectorite-coated mica according to Test Example 2 and hectorite within the range $2\theta = 2°$ to $12°$.

FIG. 17 illustrates X-ray diffraction patterns of hectorite-coated mica according to Test Example 3 and hectorite within the range $2\theta = 2°$ to $12°$.

FIG. 18 illustrates X-ray diffraction patterns of hectorite-coated mica according to Test Example 4 and hectorite within the range $2\theta = 2°$ to $12°$.

FIG. 19 illustrates X-ray diffraction patterns of hectorite-coated mica according to Test Example 5 and hectorite within the range $2\theta = 2°$ to $12°$.

FIG. 20 illustrates X-ray diffraction patterns of hectorite-coated mica according to Test Example 6 and hectorite within the range $2\theta = 2°$ to $12°$.

FIG. 21 illustrates an X-ray diffraction pattern of synthetic mica.

FIG. 22 illustrates an X-ray diffraction pattern of hectorite.

FIG. 23 is an SEM image of hectorite-coated mica according to Test Example 1.

FIG. 24 is an SEM image of hectorite-coated mica according to Test Example 1.

FIG. 25 is an SEM image of hectorite-coated mica according to Test Example 1.

FIG. 26 is an SEM image of hectorite-coated mica according to Test Example 2.

FIG. 27 is an SEM image of hectorite-coated mica according to Test Example 2.

FIG. 28 is an SEM image of hectorite-coated mica according to Test Example 2.
FIG. 29 is an SEM image of hectorite-coated mica according to Test Example 3.
FIG. 30 is an SEM image of hectorite-coated mica according to Test Example 3.
FIG. 31 is an SEM image of hectorite-coated mica according to Test Example 3.
FIG. 32 is an SEM image of hectorite-coated mica according to Test Example 4.
FIG. 33 is an SEM image of hectorite-coated mica according to Test Example 4.
FIG. 34 is an SEM image of hectorite-coated mica according to Test Example 4.
FIG. 35 is an SEM image of hectorite-coated mica according to Test Example 5.
FIG. 36 is an SEM image of hectorite-coated mica according to Test Example 5.
FIG. 37 is an SEM image of hectorite-coated mica according to Test Example 5.
FIG. 38 is an SEM image of synthetic mica.
FIG. 39 is an SEM image of synthetic mica.
FIG. 40 is an SEM image of synthetic mica.
FIG. 41 is an approximate straight line obtained in Test Example 5.
FIG. 42 is a theoretical curve of an adsorption isotherm obtained in Test Example 5.
FIG. 43 illustrates photographs showing states wherein a product according to a test example is immersed in an aqueous solution of methylene blue, and a photograph of powder separated after immersion.
FIG. 44 is an SEM image of a treated product according to Test Example 7.
FIG. 45 is an SEM image of a treated product according to Test Example 7.
FIG. 46 is an SEM image of a treated product according to Test Example 8.
FIG. 47 is an SEM image of a treated product according to Test Example 8.
FIG. 48 is a photograph of a halo test in Test Example 9-1.
FIG. 49 is a photograph of a halo test in Test Example 9-2.
FIG. 50 is a photograph of a halo test in Test Example 12-1.
FIG. 51 is a photograph of a halo test in Test Example 12-2.
FIG. 52 is a photograph of a halo test in Test Example 13-1.
FIG. 53 is a photograph of a halo test in Test Example 13-2.
FIG. 54 is a photograph of a colored silicate-coated body according to Test Example 14.
FIG. 55 is a photograph of a colored silicate-coated body according to Test Example 15.
FIG. 56 is a photograph of a colored silicate-coated body according to Test Example 16.
FIG. 57 is a photograph of a colored silicate-coated body according to Test Example 17.
FIG. 58 is a photograph of a colored silicate-coated body according to Test Example 18.
FIG. 59 is a photograph of a colored silicate-coated body according to Test Example 19.
FIG. 60 is a photograph showing mixed liquids in a state left standing before centrifugal separation/dehydration in respective coloring processes of Test Example 19 and a comparative example.

Description of Embodiments

Preferred modes of the various aspects of the present disclosure will be described below.
According to a preferred mode of the first aspect, at least a portion of the first silicate is joined to the mica particle via silica and/or a silica modified product.
According to a preferred mode of the first aspect, a median particle size of the mica particle is from 0.1 $\mu$m to 10 mm.
According to a preferred mode of the first aspect, the silicate-coated body further comprises an intermediate layer coating the mica particle. At least a portion of the first silicate coats the mica particle via the intermediate layer.
According to a preferred mode of the first aspect, the intermediate layer is a coating that exhibits an interference color.
According to a preferred mode of the first aspect, the intermediate layer comprises a metal oxide having a refractive index of 2 or greater.
According to a preferred mode of the first aspect, the intermediate layer comprises titanium dioxide, iron oxide, or a combination thereof.
According to a preferred mode of the second aspect, the substrate comprises particles of a second silicate.
According to a preferred mode of the second aspect, the substrate is flaky and/or platy mica powder.
According to a preferred mode of the second aspect, the first silicate is integral with the silica and/or the silica modified product.
According to a preferred mode of the second aspect, the silicate-coated body further comprises an intermediate layer coating the substrate. The silica and/or silica modified product adheres to at least part of a surface of the intermediate layer. At least a portion of the first silicate coats the substrate via the intermediate layer and/or the silica and/or silica modified product.

According to a preferred mode of the second aspect, the intermediate layer is a coating that exhibits an interference color.

According to a preferred mode of the second aspect, the intermediate layer comprises a metal oxide having a refractive index of 2 or greater.

According to a preferred mode of the second aspect, the intermediate layer comprises titanium dioxide, iron oxide, or a combination thereof.

According to a preferred mode of the first and second aspects, the first silicate comprises a smectite-group silicate.

According to a preferred mode of the first and second aspects, the smectite-group silicate comprises hectorite.

According to a preferred mode of the first and second aspects, the functional substance is ionic.

According to a preferred mode of the first and second aspects, the functional substance comprises at least one selected from the group consisting of antibacterial substances, bactericidal substances, sterilizing substances, and disinfecting substances.

According to a preferred mode of the first and second aspects, the functional substance is at least one selected from the group consisting of metal ions, ionic metal complexes, and cationic surfactants.

According to a preferred mode of the first and second aspects, the functional substance is at least one selected from the group consisting of a silver ion, a zinc ion, a copper ion, a diamminesilver ion, a benzalkonium ion, a benzethonium ion, a tetraethylammonium ion, and a didecyldimethylammonium ion.

According to a preferred mode of the first and second aspects, the silicate-coated body further comprises a multivalent cation. The functional substance comprises an anionic functional substance and/or an amphoteric functional substance.

According to a preferred mode of the first and second aspects, the silicate-coated body further comprises an ionic organic colorant adhering to the first silicate.

According to a preferred mode of the first and second aspects, the silicate-coated body further comprises a multivalent cation. The ionic organic colorant comprises an anionic organic colorant and/or an amphoteric organic colorant.

According to a preferred mode of the first and second aspects, the multivalent cation is at least one selected from the group consisting of a magnesium ion, a calcium ion, an aluminum ion, and a barium ion.

According to a preferred mode of the first and second aspects, the ionic organic colorant includes at least one selected from the group consisting of amaranth, new coccine, phloxine B, rose bengal, acid red, tartrazine, sunset yellow, fast green, brilliant blue, indigo carmine, lithol rubine B, lithol rubine BCA, methylene blue, rhodamine B, and erythrosine B.

In the following description, reference signs in the drawings are provided for the understanding of the invention and are not intended to limit the invention to the aspects shown. The drawings are for facilitating the understanding of silicate-coated bodies according to the present disclosure, and are not intended to limit the silicate-coated bodies to aspects illustrated in the drawings, such as illustrated shapes, dimensions, and scales. In each embodiment, the same components are accompanied by the same reference signs.

A silicate-coated body according to a first embodiment of the present disclosure will be described.

A silicate-coated body according to the present disclosure contains: a substrate; a first silicate coating at least part of the substrate; and a functional substance carried by the first silicate. In the present disclosure, the term "functional substance" may refer either to the form of a compound, to the form of a salt before ionization, or to the form of an ion after electrolytic dissociation.

Preferably, the substrate is capable of tolerating the conditions for producing the first silicate. The substrate is preferably a material to which the below-mentioned adhesive agent is adherable physically and/or chemically. The substrate preferably has a size that allows the substrate to be placed in a reaction vessel for producing the first silicate. The substrate may take the form of powder.

In cases where the substrate is powder, substrate particles may take any of various shapes, such as a spherical shape, an ellipsoidal (spheroidal) shape, a flaky shape, a platy shape, and an indefinite shape. The size of the substrate particles is preferably larger than that of the below-mentioned adhesive agent. The size of the substrate particles may be 0.1 $\mu$m or greater, 2 $\mu$m or greater, 5 $\mu$m or greater, or 7 $\mu$m or greater. The size of the substrate particles may be 10 mm or less, 1 mm or less, 500 $\mu$m or less, 200 $\mu$m or less, 100 $\mu$m or less, 50 $\mu$m or less, 40 $\mu$m or less, 30 $\mu$m or less, or 25 $\mu$m or less. The size of the substrate particles is preferably in terms of median particle size (i.e., the median of particle sizes). The median particle size can be measured, for example, by laser diffraction particle size distribution measurement.

In cases where the substrate particles are in a flaky shape or a platy shape, the average thickness of the substrate particles may be 0.05 $\mu$m or greater, 0.1 $\mu$m or greater, or 0.3 $\mu$m or greater. The average thickness of the substrate particles may be 2 $\mu$m or less, 1 $\mu$m or less, 0.5 $\mu$m or less, or 0.3 $\mu$m or less. Although methods for measuring the average thickness are not particularly limited, for example, the thicknesses of an arbitrary number of particles may be measured by inclined observation with an electron microscope, and the average thickness may be calculated as the average value thereof.

The aspect ratio of the substrate particles (median particle size/average thickness) may be 10 or greater, preferably 50 or greater, more preferably 70 or greater. The aspect ratio of the substrate particles may be 150 or less, preferably 100 or less, more preferably 90 or less. Although methods for determining the aspect ratio are not particularly limited, for example, the particle sizes and thicknesses of an arbitrary number of particles, as determined by inclined observation with an electron microscope, may be measured, and the aspect ratio may be calculated by dividing the value of the obtained median particle size by the value of the average thickness.

Examples of the substrate may include, although not limited to, a second silicate, aluminum oxide (alumina), and glass. The second silicate may be a layered silicate (phyllosilicate) different from the first silicate. Preferably, the second silicate is not swellable in water. Examples of the second silicate may include, although not limited to, mica (isinglass) and talc.

Mica that may be used as the substrate will be described in detail. Mica may be natural mica and/or synthetic mica. It is preferred to use synthetic mica from the viewpoint of chemical stability, little impurities, and plane smoothness. Examples of the synthetic mica may include, although not limited to, potassium phlogopite $[KMg_3(AlSi_3O_{10})F_2]$, potassium tetrasilisic mica $[KMg_{2\,1/2}(Si_4O_{10})F_2]$, potassium taeniolite $[KMg_2Li(Si_4O_{10})F_2]$, sodium phlogopite $[NaMg_3(AlSi_3O_{10})F_2]$, sodium taeniolite $[NaMg_2Li(Si_4O_{10})F_2]$, sodium tetrasilisic mica $[NaMg_{2\,1/2}(Si_4O_{10})F_2]$, and sodium hectorite $[Na_{1/3}Mg_{2\,2/3}Li_{1/3}(Si_4O_{10})F_2]$.

Synthetic mica used herein may be obtained by any production method, such as a melting method, a hydrothermal method, or a solid-solid reaction method. For example, synthetic mica powder can be obtained by: mixing, at a given ratio, compounds containing potassium, sodium, magnesium, aluminum, silicon, fluorine, and the like; subjecting the mixture to melting, crystallization, and cooling; and then subjecting the crystal to mechanical pulverization, heat treatment, washing, and drying. For example, in the case of synthetic fluorophlogopite (potassium phlogopite), synthetic fluorophlogopite can be obtained by: weighing and mixing silicic acid anhydride, magnesium oxide, aluminum oxide, and potassium silicofluoride so that the mixture has the above composition; melting the mixture at 1,400 to 1,500°C; and cooling the same to room temperature. Synthetic mica powder can be obtained by pulverizing a lump of the obtained synthetic fluorophlogopite, and classifying the particles as needed.

The first silicate may coat part of the substrate, or may coat the whole substrate. The first silicate may contain a smectite-group silicate. The smectite-group silicate may be, for example, hectorite. An ideal composition of hectorite can be expressed as $[Li_x(Mg_{6-x}Li_xSi_8O_{20}(OH)_4 \cdot nH_2O)]$.

The thickness of the first silicate on the surface of the substrate may be 5 nm or greater, preferably 10 nm or greater. The thickness of the first silicate on the surface of the substrate may be 100 nm or less, preferably 50 nm or less. The thickness of the first silicate can be determined with a transmission electron microscope (TEM).

The content by percentage of the first silicate in the silicate-coated body may be 10% by mass or greater, or 15% by mass or greater, relative to the mass of the silicate-coated body. The content by percentage of the first silicate may be 30% by mass or less, or 25% by mass or less, relative to the mass of the silicate-coated body.

The content by percentage of the first silicate in the silicate-coated body can be calculated, for example, from the Langmuir adsorption isotherm. The Langmuir adsorption isotherm can be expressed as Math. 1. In Math. 1, q is the amount of adsorbed colorant, $q_m$ is the maximum colorant adsorption amount (saturated adsorption amount), K is the equilibrium constant, and C is the concentration of added colorant (equilibrium concentration). First, a certain amount (e.g., x grams) of the first silicate (e.g., hectorite) and a colorant (e.g., methylene blue) are mixed in water, and the colorant adsorption amount q based on the first silicate in the supernatant liquid is calculated. This is repeated while varying the colorant addition amounts C, to find the respective colorant adsorption amounts q relative to the colorant addition amounts C. Math. 1 can be changed to Math. 2. The measured values are plotted according to Math. 2, wherein the horizontal axis indicates the colorant addition amount C and the vertical axis indicates the ratio, C/q, of the colorant addition amount C to the adsorption amount q, to find the first maximum colorant adsorption amount $q_m$ and the first equilibrium coefficient K for the first silicate from the slope $(1/q_m)$ and the intercept $(1/q_mK)$. Next, in the same way, the colorant addition amounts C and the colorant adsorption amounts q are measured for a certain amount (e.g., x grams) of the silicate-coated body of the present disclosure (i.e., for the first silicate contained in the silicate-coated body), instead of the first silicate, to find the second maximum colorant adsorption amount $q_m$ and the second equilibrium coefficient K. The content by percentage of the first silicate in the silicate-coated body can be calculated by comparing the first maximum colorant adsorption amount $q_m$ and the second maximum colorant adsorption amount $q_m$.

[Math. 1]

$$q = \frac{q_m \cdot K \cdot C}{1 + K \cdot C}$$

[Math. 2]

$$\frac{C}{q} = \frac{1}{q_m} \cdot C + \frac{1}{q_m \cdot K}$$

The first silicate can be synthesized on the surface of the substrate as described in the below-mentioned manufacturing method. In cases where the composition, constitution, properties or the like of the first silicate cannot be directly specified, the first silicate can be defined according to the manufacturing method.

The functional substance is preferably a substance that dissolves in water in the form of ions. The functional substance may be an inorganic compound or an organic compound. The functional substance may be a cationic substance, an anionic substance, an amphoteric substance, an acidic substance, and/or a basic substance. It is thought that the functional substance is contained in the first silicate. It is thought that the functional substance forms a complex with the first silicate. It is thought that the functional substance is adsorbed by the first silicate by ionic interaction and/or electrostatic interaction.

Examples of functional substances usable herein may include, although not limited to, substances having, e.g., antibacterial, bactericidal, sterilizing, disinfectant, or other actions (e.g., antibacterial agents, bactericides, sterilizing agents, or disinfectants). Examples of such functional substances may include, although not limited to: metal ions such as silver, zinc and copper ions; ionic metal complexes containing a metal ion such as a silver, zinc, or copper ion (e.g., a diamminesilver ion); and cationic surfactants such as quaternary ammonium salts (e.g., a benzalkonium ion, a benzethonium ion, a tetraethylammonium ion, and a didecyldimethylammonium ion).

In cases where the functional substance is an anionic substance and/or an amphoteric substance, the silicate-coated body further contains a multivalent ion. The multivalent ion may be a di- or higher valent cation. Examples of the multivalent ion may include, although not limited to, alkaline-earth metal ions and metal ions. Examples of the multivalent cation may include, although not limited to, a magnesium ion ($Mg^{2+}$), calcium ion ($Ca^{2+}$), aluminum ion ($Al^{3+}$), and barium ion ($Ba^{2+}$). Other examples of multivalent cations may include complex ions such as a hexaaquaaluminum ion ($[Al(H_2O)_6]^{3+}$).

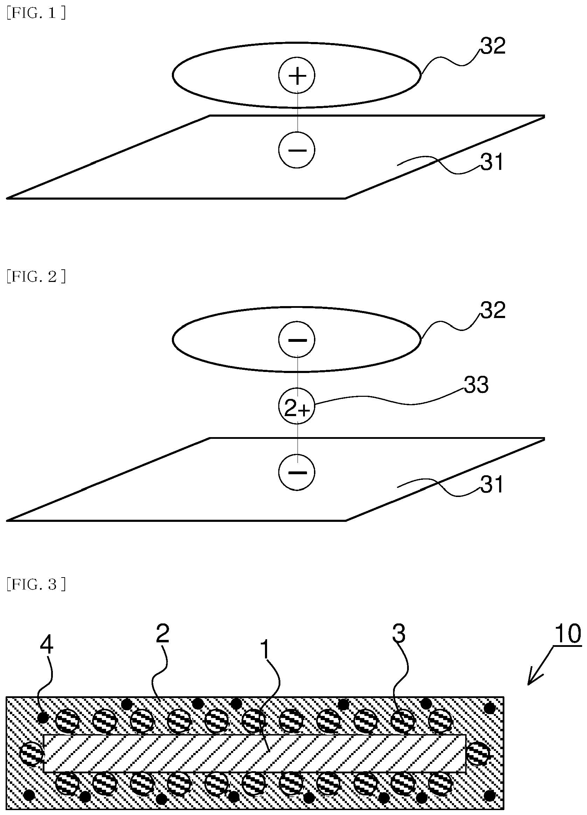

A structure of the silicate-coated body according to the present disclosure will be described below, taking, as an example, a case where the first silicate is a silicate having a layered structure like a smectite-group silicate. FIG. 1 and FIG. 2 show conceptual drawings of silicate-coated bodies according to the present disclosure. FIG. 1 is a conceptual drawing in cases where the functional substance is a cationic substance. FIG. 2 is a conceptual drawing in cases where the functional substance is an anionic substance and/or an amphoteric substance. Note, however, that, even if the structures shown below differ from actual structures, the actual structures are not excluded from the scope of the present disclosure.

As shown in FIG. 1, in cases where the functional substance 32 is a cationic substance, it is thought that the functional substance 32 is incorporated into the first silicate by ionic exchange with an exchangeable positive ion in the smectite-group silicate. It is also thought that the functional substance 32 is adsorbed by the first silicate by the ionic/electrostatic interaction between an ionic moiety of the functional substance 32 and a sheet structure 31 of the first silicate.

As shown in FIG. 2, in cases where the functional substance 32 is an anionic substance and/or an amphoteric substance, it is thought that the functional substance 32 is incorporated into the first silicate via a multivalent cation 33. Since the functional substance 32 has the same charge as the sheet structure 31 of the first silicate, the functional substance 32 cannot be incorporated into the first silicate by direct ionic exchange with an exchangeable positive ion in the smectite-group silicate. It is therefore thought that, by interposing the multivalent cation 33 having a charge opposite to that of the sheet structure 31 between the functional substance 32 and the sheet structure 31 of the first silicate, the functional substance 32 is adsorbed by the first silicate by the ionic/electrostatic interaction among the ionic functional group of the functional substance 32, the multivalent cation 33, and the sheet structure 31 of the first silicate.

Since the charge of the multivalent cation 33 needs to be theoretically equivalent to the charge of the sheet structure 31 of the first silicate and the charge of the ionic functional group of the functional substance 322 opposing the sheet structure 31 (or the charge of the whole functional substance), the charge of the multivalent cation 33 needs to have a valence of two or more (e.g., divalent, trivalent, or the like).

The content by percentage of the functional substance can be suitably set depending on the intended use, properties, and the like.

In cases where the functional substance is a cationic substance, the content by percentage of the functional substance relative to the mass of the silicate-coated body may be, for example, 0.05% by mass or greater, 0.1% by mass or greater, 0.5% by mass or greater, 1% by mass or greater, 3% by mass or greater, or 5% by mass or greater. The content by percentage of the functional substance relative to the mass of the silicate-coated body may be, for example, 15% by mass or less, 12% by mass or less, or 10% by mass or less.

In cases where the functional substance is an anionic substance, the content by percentage of the functional substance relative to the mass of the silicate-coated body may be, for example, 0.05% by mass or greater, 0.1% by mass or greater, 0.5% by mass or greater, 1% by mass or greater, 3% by mass or greater, or 5% by mass or greater. The content by percentage of the functional substance relative to the mass of the silicate-coated body may be, for example, 10% by mass or less, 8% by mass or less, or 5% by mass or less.

The content by percentage of the multivalent cation can be suitably set according to the content by percentage of the anionic substance. The content by percentage of the multivalent cation relative to the mass of the silicate-coated body may be, for example, 0.1% by mass or greater, 0.5% by mass or greater, or 1% by mass or greater. The content by percentage of the multivalent cation relative to the mass of the silicate-coated body may be, for example, 10% by mass or less, 8% by mass or less, or 6% by mass or less.

The amount of functional substance adsorbed by the silicate-coated body can be measured, for example, by inductively coupled plasma-mass spectrometry (ICP-MS).

There are cases where, like hectorite, the first silicate alone may be minute. Even in such cases, according to the silicate-coated body of the first embodiment, the first silicate can be handled at the size of the substrate while exhibiting the effect of the first silicate on the substrate's surface, thereby improving handleability. Further, the usability of the first silicate can be improved, compared to cases where the first silicate is used alone.

For example, in cases where the first silicate is a smectite-group silicate, the silicate-coated body will be able to carry(support) functional substances through ion exchange. Particularly, even in cases where the interlayer ion (e.g., potassium) of the substrate (e.g., mica) has low ion exchangeability and thus the ion exchangeability of the substrate is low, the substrate will be able to carry a functional substance. Thus, the silicate-coated body can exhibit functions/actions ascribable to the carried functional substance. For example, in cases where an antibacterial substance is carried, the silicate-coated body can exhibit antibacterial actions.

According to the silicate-coated body of the first embodiment, by selecting the substrate, the surface area can be enlarged as compared with that of the first silicate alone. For example, in cases where the silicate-coated body is used as a cationic adsorbent, the adsorption efficiency can therefore be enhanced as compared with that of the first silicate alone. Also, the silicate-coated body can be collected easily after the silicate-coated body adsorbs an object.

According to the silicate-coated body of the first embodiment, the shape of the first silicate can be diversified by selecting the substrate. For example, in cases where a platy or flaky substrate is selected, the first silicate can also be used substantially in a platy or flaky form.

According to the first embodiment, aggregation of the silicate-coated body can be suppressed. Thus, the functional substance can be carried uniformly by the silicate-coated body. The silicate-coated body has excellent texture attributable to the substrate (e.g., mica-group silicate), and also has excellent handleability. Thus, the silicate-coated body is applicable to various uses.

The silicate-coated body of the first embodiment can be imparted with both the functions of the first silicate and those of the substrate. Alternatively, the function of the substrate can be adjusted or improved with the first silicate. Mutually employing the functions of both the substrate and the first silicate can serve to adjust, expand, and/or improve the functions of the substrate and/or the first silicate.

The following describes an example of hectorite-coated mica powder, wherein the substrate is phlogopite powder and the first silicate is hectorite. In hectorite-coated mica powder, cation exchangeability, which phlogopite alone cannot achieve, can be imparted to phlogopite. Hectorite-coated mica powder will be able to adsorb, for example, a different type of metal cation, organic cation, or metal oxide. By using this function, the color tone of phlogopite can be changed with coloring ions, and new functions can be imparted to phlogopite with functional ions. The utilization of these functions will enable the powder to be utilized as colored platy pigments and functional platy powders in cosmetic applications and industrial applications. Functionality may be imparted, for example, by exchanging an ion in hectorite with a different type of metal cation or a metal oxide; in this way, a film having a different refractive index can be produced on the surface of phlogopite, to impart such properties as design aesthetics. Meanwhile, by coating phlogopite with hectorite, the volume and the specific surface area can be increased as compared with those of phlogopite alone, thereby increasing oil absorptivity. By this improvement in oil absorptivity, smearing of makeup by sebum can be suppressed, and also the amount of oily components added to cosmetics can be increased. By coating phlogopite with hectorite to change the properties and condition of the surface of the powder, light reflectivity/diffusibility can be adjusted.

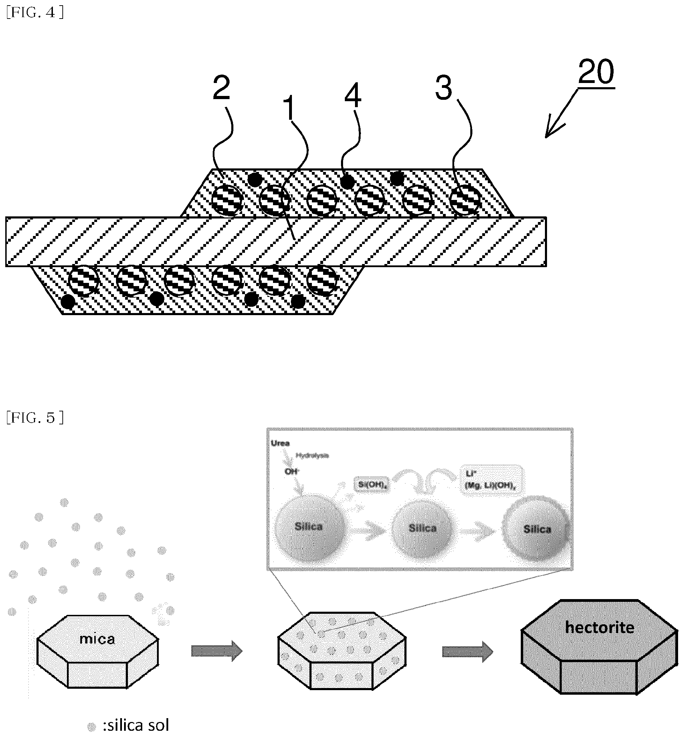

A silicate-coated body according to a second embodiment of the present disclosure will be described. FIG. 3 and FIG. 4 show schematic sectional views of silicate-coated bodies according to the second embodiment.

Silicate-coated bodies 10 and 20 of the present disclosure further contain an adhesive agent 3, in addition to the substrate 1, the first silicate 2 and the functional substance 4 in the first embodiment. The adhesive agent 3 may exist on the substrate 1. At least a portion of the first silicate 2 may coat the substrate 1 via the adhesive agent 3. The first silicate 2 may exist along the arrangement of the adhesive agent 3. It is preferred that the adhesive agent 3 is capable of making the first silicate 2 adhere to the substrate 1. The adhesive agent 3 is preferably a raw material for synthesizing the first silicate. The first silicate 2 is preferably formed integrally with the adhesive agent 3.

The adhesive agent 3 is preferably, for example, silica and/or a silica modified product. The silica and/or silica modified product may also include a compound in which the surface of silica has been modified. The silica modified product may include compounds derived from silica, compounds generated from silica in a reaction process, or the like. The silica and/or silica modified product will be simply referred to as "silica" hereinafter.

Silica preferably takes the form of powder. A silica particle is preferably smaller than the substrate so that it can adhere to the surface of the substrate. The average particle size of silica particles is preferably 50 nm or smaller, more preferably 30 nm or smaller, and further preferably 20 nm or smaller. It is thought that, if the average particle size is greater than 50 nm, silica is hard to adhere to the surface of the substrate, thereby making it difficult to produce hectorite on the surface of the substrate.

In terms of the ratio of the size of the silica particle to the size of the substrate, assuming that the average particle size of the silica particles is 1, it is preferred that the median particle size of the substrate is 10 or greater, more preferably 50 or greater, further preferably 100 or greater. This is because increasing the size of the silica particles relative to the substrate will reduce the number of silica particles adhering to the substrate, thereby leading to a reduction in the amount of the first silicate coating.

The silicate-coated body according to the second embodiment can also have the same effects as the silicate-coated body according to the first embodiment. The existence of the adhesive agent enables enhancement of the joining ability between the first silicate and the substrate.

Some characteristics other than the above in the silicate-coated body of the present disclosure are difficult to directly specify by the structure or properties of the silicate-coated body of the present disclosure. In such cases, it is useful to specify the characteristics by the below-mentioned manufacturing method. For example, in cases where, for example, the form, composition, existence, distribution, or content by percentage of the adhesive agent cannot be directly specified, it is useful to specify these by the below-mentioned manufacturing method.

As a third embodiment of the present disclosure, a method for manufacturing the silicate-coated body according to the first and second embodiments will be described. FIG. 5 shows a schematic diagram for describing a structure of a silicate-coated body according to the second embodiment, and a mechanism for producing the same. The method described below is one aspect, and the method for manufacturing the silicate-coated body of the present disclosure is not limited to the following manufacturing method. The reaction mechanism included in the following description is complementary, and it is not intended to limit the manufacturing method of the present disclosure. That is, even if an actual reaction mechanism proves to be different from the below-mentioned mechanism, that will not influence the following manufacturing method.

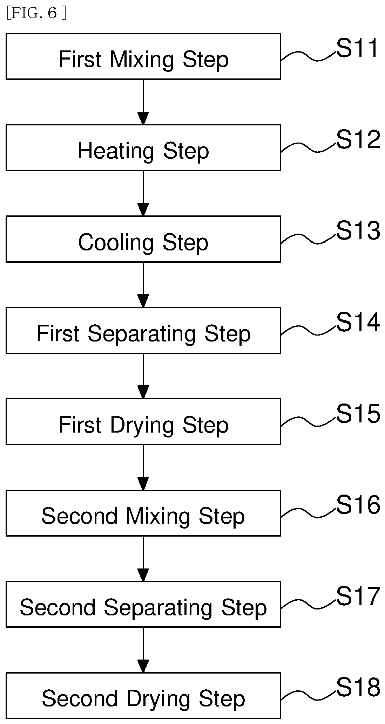

FIG. 6 shows a flowchart of the manufacturing method according to the third embodiment.

A mixed liquid is prepared by adding, to a solvent, a raw material containing elements constituting a first silicate, a dissolving agent which dissolves at least a portion of the raw material, and a substrate (S11; first mixing step). As the solvent, for example, water may be used. It is preferred to apply ultrasonic waves to the mixed liquid to disperse the added ingredients in the solvent.

For the substrate, the substrate described in the first embodiment may be used. The substrate preferably has a surface to which silica (silicon dioxide; silicic acid anhydride; $SiO_2$) particles are adherable.

The raw material containing the elements constituting the first silicate may include silica powder (which may take the sol form and/or the gel form). The silica powder serves as a raw material of the first silicate, and can also function as a starting point for coating the substrate with the first silicate. In cases where the first silicate is a smectite-group silicate such as hectorite, the raw material preferably contains a lithium compound, a magnesium compound, or the like.

The shape of silica particles is not particularly limited. For example, the silica particles may have, for example, a spherical shape, a platy shape, a scaly shape, or an indefinite shape. Silica may be a porous body or may be a non-porous body. The surface of silica is preferably hydrophilic.

The size of silica particles is preferably smaller than the size of the substrate (including substrate particles) so that the silica particles can adhere to the surface of the substrate. In terms of the ratio of the size of the silica particle to the size of the substrate, assuming that the average particle size of the silica particles is 1, it is preferred that the median particle size of the substrate is 10 or greater, more preferably 50 or greater, further preferably 100 or greater. This is because, if the size of the silica particles is increased relative to the substrate, the number of silica particles that can adhere to the substrate will be reduced, thereby leading to a reduction in the amount of the first silicate coating.

The particle size of the silica particles can be set as appropriate, depending on the design of the surface area of silicate-coated powder. The average particle size of the silica powder may be, for example, 5 nm or greater, or 10 nm or greater. The average particle size of the silica powder may be, for example, 2 $\mu$m or less, 1 $\mu$m or less, 500 nm or less, 200 nm or less, 100 nm or less, 50 nm or less, or 20 nm or less.

The mixing ratio of the silica powder, with respect to 1 part by mass of the substrate, is preferably 0.02 parts by mass or greater, more preferably 0.05 parts by mass or greater, further preferably 0.08 parts by mass or greater, further preferably 0.1 parts by mass or greater, further preferably 0.15 parts by mass or greater. If the mixing ratio is less than 0.02 parts by mass, the formation of smectite becomes insufficient. The mixing ratio of the silica powder is preferably 0.7 parts by mass or less, more preferably 0.5 parts by mass or less, further preferably 0.3 parts by mass or less, further preferably 0.25 parts by mass or less. If the mixing ratio is greater than 0.7 parts by mass, the substrate aggregates and thus is difficult to be coated with hectorite.

The lithium compound may be any lithium compound which can be a raw material of a lithium element contained in a smectite. Examples of the lithium compound usable herein may include, although not limited to, lithium fluoride (LiF) and lithium chloride (LiC1).

The magnesium compound may be any magnesium compound which can be a raw material of a magnesium element contained in a smectite. Examples of the magnesium compound usable herein may include, although not limited to, magnesium chloride (MgCh), magnesium hydroxide (Mg(OH)$_2$), and magnesium oxide (MgO).

The dissolving agent is preferably a compound which can dissolve surface portions of the silica particles. Examples of the dissolving agent usable herein may include, although not limited to, sodium hydroxide (NaOH) and compounds capable of producing hydroxide ions (OH$^-$) by hydrolysis, such as urea (CO(NH$_2$)$_2$).

Next, the mixed liquid is heated (S12; heating step). The mixed liquid is preferably heated while being pressurized. For example, the mixed liquid can be heated and pressurized with an autoclave. Preferably, the mixed liquid is heated, for example, at a temperature of 80°C or higher, preferably 100°C or higher, for 30 hours or more, preferably 40 hours or more.

The reaction product is cooled after heating (S13; cooling step). Cooling is preferably performed by rapid cooling. The solid content in the reaction product is separated after cooling (S14; first separating step). Separation may be performed by centrifugal separation treatment or the like. Next, the separated product is dried (S15; first drying step), and thereby a silicate-coated body by which the functional substance is not carried can be obtained. Other steps, such as a washing step, may be included as appropriate.

In cases where the silicate-coated body is not isolated, the first separating step (S14) and the first drying step (S15) do not need to be performed.

FIG. 3 shows a process in which the surface of a mica particle as a substrate is coated with hectorite. The coating mechanism of hectorite is thought to be as follows. First, silica particles adhere to the surface of a mica particle. Next, hydroxide ions produced by hydrolyzing urea, which is a dissolving agent, attack the silica particles adhering to the surface of the mica particle. The outer layer of the silica particles is dissolved by this attack, thereby producing a silicon compound. The silicon compound produced by the dissolution of the outer layer of silica reacts with the lithium compound and the magnesium compound which are added as raw materials. This forms hectorite on the surface of the silica particles, thereby coating the mica particle with hectorite.

Whether the first silicate has been produced on the surface of the substrate can be verified, for example, by X-ray diffraction measurement. The production of the first silicate can be also verified by whether the product can be colored with a cationic colorant (e.g., methylene blue).

An aqueous solvent including water, the silicate-coated body produced in S15, and a functional substance are mixed (S16; second mixing step). The aqueous solvent may be an aqueous solvent that can electrolytically dissociate the functional substance and that does not inhibit the adsorption of the functional substance to the silicate-coated body. The silicate-coated body and the functional substance may be added in any order or simultaneously. An aqueous solution in which the functional substance is dissolved in water separately may be added to a dispersion medium of the silicate-coated body. It is thought that the functional substance is ionized in the aqueous solvent. As the functional substance, any of the aforementioned colorants may be used. One or more types of the functional substance may be used.

The rate of the silicate-coated body added can be suitably set. The rate of the functional substance added can be suitably set depending on the desired use/application and characteristics.

In cases where the functional substance is an anionic substance, a salt or a compound (multivalent cation source) which can produce multivalent cations by electrolytic dissociation is dissolved in the aqueous solvent. Examples of the multivalent cation source may include, although not limited to, chlorides and hydroxides of multivalent cations. Examples of the multivalent cation source may include, although not limited to, calcium chloride (CaCh), magnesium chloride (MgCh), barium chloride (BaCh), and aluminum chloride hydrate ([Al(H$_2$O)$_6$]Cl$_3$). One or more types of the multivalent cation source may be used.

The aqueous solvent containing the silicate-coated body and the functional substance is preferably stirred to increase the carrying speed.

The amount of the functional substance added can be suitably set depending on the intended use/application. The proportion of the functional substance added relative to 100 parts by mass of a non-carrying silicate-coated body added in S16 may be, for example, 0.01 parts by mass or more, 0.1 parts by mass or more, 0.2 parts by mass or more, or 0.5 parts by mass or more. The proportion of the functional substance added relative to 100 parts by mass of a non-carrying silicate-coated body may be, for example, 2 parts by mass or less, 1.5 parts by mass or less, or 1 part by mass or less.

The amount of the multivalent cation source added can be suitably set depending on the amount of the anionic substance added. The proportion of the multivalent cation source added relative to 100 parts by mass of the non-carrying silicate-coated body added in S16 may be, for example, 0.5 parts by mass or more, 1 part by mass or more, or 2 parts by mass or more. The proportion of the multivalent cation source added relative to 100 parts by mass of the non-carrying silicate-coated body may be, for example, 12 parts by mass or less, 10 parts by mass or less, or 8 parts by mass or less.

Next, the silicate-coated body carrying the functional substance is separated from the aqueous solvent by filtration or the like (S17; second separating step). Next, the separated silicate-coated body is dried (SI8; second drying step). A silicate-coated body can be obtained thereby. In cases where the silicate-coated body does not need to be isolated, the second separating step (S17) and the second drying step (S18) do not have to be performed. Other steps, such as a washing step, may be included as appropriate.

Typically, a substrate (e.g., mica) itself cannot be made to carry a functional substance simply by mixing the substrate and the functional substance. However, according to the manufacturing method of the third embodiment, even a substrate which has difficulty in carrying a functional substance directly can be made to carry a functional substance by a simple method.

According to the manufacturing method of the third embodiment, the substrate can carry a functional substance regardless of whether the functional substance is anionic or cationic. The silicate-coated body can be provided with desired functions by selection and combination of the functional substances.

According to the manufacturing method of the third embodiment, the silicate-coated bodies according to the first embodiment and the second embodiment can be manufactured. Even in cases where there is no adhesiveness or joining ability between the first silicate and the substrate, the manufacturing method of the present disclosure can coat the substrate with the first silicate. Even in cases where the substrate is powder, the substrate can be coated with the first silicate at the particle level. The manufacturing method of the present disclosure can increase the degree of freedom in designing the first silicate, and can also expand the use thereof.

As a fourth embodiment of the present disclosure, a manufacturing method for manufacturing the silicate-coated bodies according to the first and second embodiments will be described. In the third embodiment, the substrate is coated with the first silicate to produce the silicate-coated body, and then a functional substance is carried. In the fourth embodiment, the substrate is coated with the first silicate and is made to carry a functional substance at the same time.

In the fourth embodiment, a functional substance is further added in the first mixing step (S11) in the third embodiment. Stated differently, the second mixing step (S16) is executed simultaneously with the first mixing step (S11). In cases where an anionic substance is used, a salt used as a multivalent cation source is also added together. Other than the addition of the functional substance and the multivalent cation source, the method can be performed in the same way as in the third embodiment. The second separating step (S17) and the second drying step (S18) in the third embodiment may be omitted.

According to the fourth embodiment, a silicate-coated body can be obtained with more simplified steps than those of the third embodiment. The fourth embodiment is useful in cases where the functional substance can tolerate a heating step and in cases where disadvantages such as the aggregation of the functional substance do not occur.

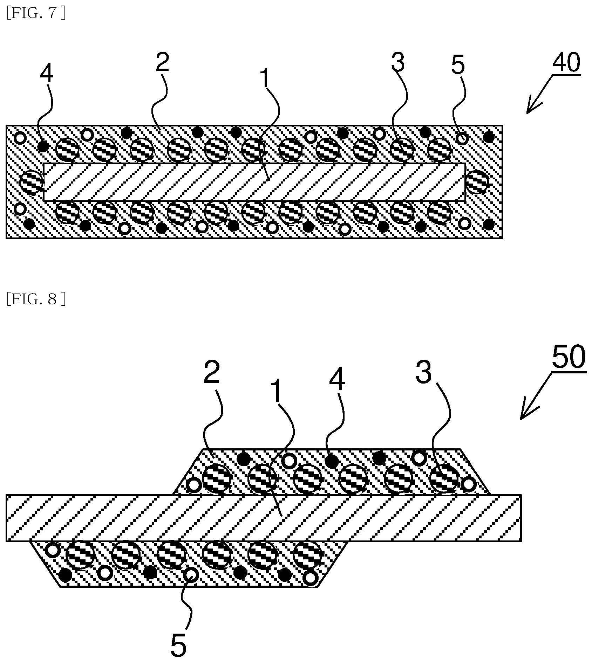

Silicate-coated bodies according to a fifth embodiment of the present disclosure will be described. The silicate-coated bodies according to the fifth embodiment relate to a colored aspect of the silicate-coated bodies according to the first embodiment, the second embodiment and the seventh embodiment. FIGS. 7 and 8 show schematic sectional views of silicate-coated bodies according to the fifth embodiment. FIGS. 7 and 8 show configurations in which the silicate-coated bodies of the second embodiment are colored. Reference can be made to the description above in the present disclosure regarding the silicate-coated bodies according to the first, second and seventh embodiments, and explanation thereon will be omitted hereinbelow. In the present disclosure, the term "ionic organic colorant" may refer either to the form of a salt before ionization or to the form of an ion after electrolytic dissociation.

The silicate-coated bodies 40, 50 according to the fifth embodiment further contain an ionic organic colorant 5, in addition to the ingredients of the first, second or seventh embodiment. "Ionic organic colorant" 5 means an organic compound that dissolves in water in the form of ions. As the ionic organic colorant 5, at least one selected from the group consisting of cationic organic colorants, anionic organic colorants, amphoteric colorants, acidic organic colorants, and a basic organic colorant can be used depending on the desired color. It is thought that the ionic organic colorant 5 is contained in the first silicate 2. It is thought that the ionic organic colorant 5 forms a complex with the first silicate 2. It is thought that the ionic organic colorant 5 is adsorbed by the first silicate 2 by ionic interaction and/or electrostatic interaction.

Examples of ionic organic colorants usable herein may include, although not limited to, tar colorants (statutory colorants) stipulated, for example, in "Ministerial Ordinance to Establish Tar Colors Usable in Pharmaceuticals." Examples of ionic organic colorants may include, although not limited to, one or more of colorants belonging to Group I, such as amaranth (red No. 2), erythrosine (red No. 3; tetraiodofluorescein sodium salt), new coccine (red No. 102), phloxine B (red No. 104), rose bengal (red No. 105), acid red (red No. 106), tartrazine (yellow No. 4), sunset yellow (yellow No. 5), fast green (green No. 3), brilliant blue (blue No. 1; erioglaucine A; acid blue 9), and indigo carmine (blue No. 2), and colorants belonging to Group II, such as lithol rubine B (red No. 201), lithol rubine BCA (red No. 202), and rhodamine B (red No. 213; basic violet 10).

Examples of cationic organic colorants usable herein may include, although not limited to, methylene blue and rhodamine B. Examples of anionic organic colorants usable herein may include, although not limited to, erythrosine B, tartrazine, sunset yellow FCF, brilliant blue FCF, amaranth, new coccine, phloxine B, rose bengal, indigo carmine, sunset yellow, and lithol rubine B. Examples of amphoteric organic colorants usable herein may include, although not limited to, acid red and fast green FCF.

Whether the organic colorant is cationic or anionic can be determined by the counter ion. In cases where the counter ion is an anion, the organic colorant is cationic, which has the opposite charge. In cases where the counter ion is a cation, the organic colorant is anionic, which has the opposite charge. An amphoteric colorant may carry both a positive charge and a negative charge within a molecule, thereby making the net charge of the whole molecule zero.

In cases where the ionic organic colorant is an anionic organic colorant and/or an amphoteric organic colorant, the silicate-coated body further contains a multivalent ion. The multivalent ion may be a di- or higher valent cation. Examples of the multivalent ion may include, although not limited to, alkaline-earth metal ions and metal ions. Examples of the multivalent cation may include, although not limited to, a magnesium ion ($Mg^{2+}$), calcium ion ($Ca^{2+}$), aluminum ion ($Al^{3+}$), and barium ion ($Ba^{2+}$). Other examples of multivalent cations may include complex ions such as a hexaaquaaluminum ion ($[Al(H_2O)_6]^{3+}$).

It is thought that the ionic organic colorant is carried by the first silicate according to the same configuration as the functional substance illustrated in FIG. 1 and FIG. 2. That is, the conceptual diagrams of FIGS. 1 and 2 can also be used for the present fifth embodiment, with reference number 32 instead indicating an ionic organic colorant.

The content by percentage of the ionic organic colorant can be suitably set depending on the desired color tone.

In cases where the ionic organic colorant is a cationic colorant, the content by percentage of the ionic organic colorant relative to the mass of the silicate-coated body may be, for example, 0.05% by mass or greater, 0.1% by mass or greater, 0.5% by mass or greater, 1% by mass or greater, 3% by mass or greater, or 5% by mass or greater. The content by percentage of the ionic organic colorant relative to the mass of the silicate-coated body may be, for example, 15% by mass or less, 12% by mass or less, or 10% by mass or less.

In cases where the ionic organic colorant is an anionic colorant, the content by percentage of the ionic organic colorant relative to the mass of the silicate-coated body may be, for example, 0.05% by mass or greater, 0.1% by mass or greater, 0.5% by mass or greater, 1% by mass or greater, 3% by mass or greater, or 5% by mass or greater. The content by percentage of the ionic organic colorant relative to the mass of the silicate-coated body may be, for example, 10% by mass or less, 8% by mass or less, or 5% by mass or less.

The content by percentage of the multivalent cation can be suitably set according to the content by percentage of the anionic organic colorant. The content by percentage of the multivalent cation relative to the mass of the silicate-coated body may be, for example, 0.1% by mass or greater, 0.5% by mass or greater, or 1% by mass or greater. The content by percentage of the multivalent cation relative to the mass of the silicate-coated body may be, for example, 10% by mass or less, 8% by mass or less, or 6% by mass or less.

The amount of the ionic organic colorant adsorbed by the silicate-coated body can be measured by absorption wavelength analysis by spectroscopic analysis, for example. The adsorbed amount of the colorant can be determined by comparing the peak intensity of the colored silicate-coated body with the peak intensity of a colorant solution having a prescribed concentration.

The silicate-coated body according to the fifth embodiment can achieve the same effects as those of the foregoing embodiments. The silicate-coated body according to the fifth embodiment of the present disclosure can be used, for example, as a pigment. The ionic organic colorant adsorbed by the first silicate is hard to desorb, thus making decoloration and color migration less likely. By using a highly safe ionic organic colorant, a highly safe colored silicate-coated body can be obtained. The colored silicate-coated body is less prone to aggregate, and thus can be easily used. For example, the colored silicate-coated body is therefore applicable to cosmetics and the like.

According to the silicate-coated body of the fifth embodiment of the present disclosure, the stability of the ionic organic colorant can be enhanced, and thereby fading can be suppressed. Some unadsorbed ionic organic colorants are easily decomposed by light, heat, oxygen, or the like. When decomposition of the ionic organic colorant proceeds, fading occurs. In contrast, by adsorbing the ionic organic colorant in the first silicate, the decomposition of the ionic organic colorant can be suppressed. Therefore, by using the colored silicate-coated body as an alternative to such ionic organic colorants, the durability of color strength can be enhanced.

The silicate-coated body according to the fifth embodiment of the present disclosure has high usability as a pigment. Typical dyes/pigments used for cosmetics or the like aggregate because they generally undergo a drying step. Therefore, typical dyes/pigments can only be used after the aggregates have been crushed by one of various methods at the time of use. In contrast, the silicate-coated body of the present disclosure is less prone to aggregate. Therefore, the colored silicate-coated body of the present disclosure does not require dispersing/crushing, and is therefore highly usable. With typical dyes/pigments, aggregation gives rise to a change in color strength as well as degradation in texture. In contrast, according to the silicate-coated body of the present disclosure, changes in color strength and degradation in texture can be suppressed.

By selecting the type of ionic organic colorant, the colored silicate-coated body of the present disclosure can be provided with a color that a substrate (e.g., mica) alone cannot usually have.

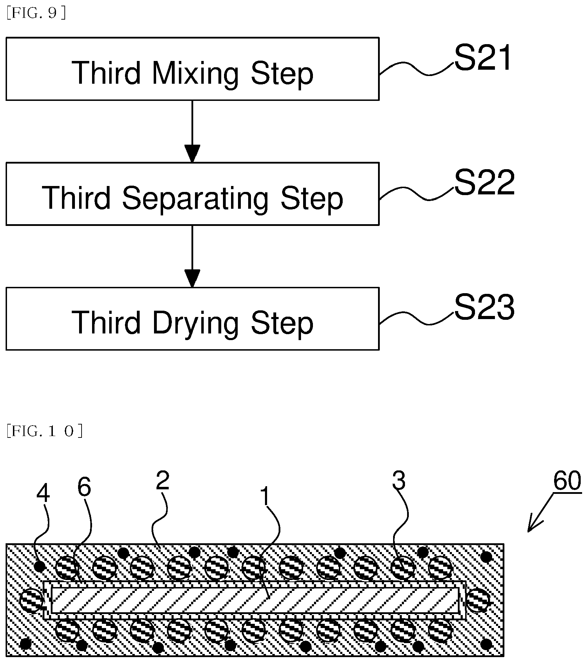

As a sixth embodiment of the present disclosure, a method for manufacturing the silicate-coated body according to the fifth embodiment will be described. FIG. 9 shows a flowchart of a manufacturing method according to the sixth embodiment.

An ionic organic colorant can be made to adhere to the first silicate in the same manner as with the functional substance in the foregoing embodiments. More specifically, the manufacturing method according to the sixth embodiment further involves, in addition to the foregoing embodiments, a step of mixing an aqueous solvent including water, a silicate-coated body according to any of the foregoing embodiments, and an ionic organic colorant (S21; third mixing step). After the third mixing step, the silicate-coated body manufacturing method according to the sixth embodiment may further involve a third separating step (S22) and a third drying step (S23), as will be described further below.

The order in which the functional substance and the ionic organic colorant adhere to the first silicate is not particularly limited, so long as the actions thereof do not inhibit one another. Stated differently, the third mixing step may be provided in any of the steps in the third and fourth embodiments, so long as it does not inhibit the other steps. For example, the steps from the third mixing step to the third drying step may be performed after the first drying step (S15), or after the second drying step (S18). Alternatively, the third mixing step may be performed simultaneously with the first mixing step (S15), or simultaneously with the second mixing step (S18).

The aqueous solvent may be an aqueous solvent that can electrolytically dissociate the ionic organic colorant and that does not inhibit the adsorption of the ionic organic colorant to the silicate-coated body. The silicate-coated body and the ionic organic colorant may be added in any order or simultaneously. An aqueous solution in which the ionic organic colorant is dissolved in water separately may be added to a dispersion medium of the silicate-coated body. It is thought that the ionic organic colorant is ionized in the aqueous solvent. As the ionic organic colorant, any of the aforementioned colorants may be used. One or more types of the ionic organic colorant may be used.

The rate of the silicate-coated body added can be suitably set. The rate of the ionic organic colorant added can be suitably set depending on the desired darkness/lightness of coloring.

In cases where the ionic organic colorant is an anionic organic colorant, a salt or a compound (multivalent cation source) which can produce multivalent cations by electrolytic dissociation is dissolved in the aqueous solvent. Examples of the multivalent cation source may include, although not limited to, chlorides and hydroxides of multivalent cations. Examples of the multivalent cation source may include, although not limited to, calcium chloride (CaCh), magnesium chloride (MgCh), barium chloride (BaCh), and aluminum chloride hydrate ($[Al(H_2O)_6]Cl_3$). One or more types of the multivalent cation source may be used.

The aqueous solvent containing the silicate-coated body and the ionic organic colorant is preferably stirred to increase the coloring speed.

The amount of the ionic organic colorant added can be suitably set depending on the desired color tone. The proportion of the ionic organic colorant added relative to 100 parts by mass of the uncolored silicate-coated body may be, for example, 0.01 parts by mass or more, 0.1 parts by mass or more, 0.2 parts by mass or more, or 0.5 parts by mass or more. The proportion of the ionic organic colorant added relative to 100 parts by mass of the uncolored silicate-coated body may be, for example, 2 parts by mass or less, 1.5 parts by mass or less, or 1 part by mass or less.

The amount of the multivalent cation source added can be suitably set depending on the amount of the anionic organic colorant added. The proportion of the multivalent cation source added relative to 100 parts by mass of the uncolored silicate-coated body may be, for example, 0.5 parts by mass or more, 1 part by mass or more, or 2 parts by mass or more. The proportion of the multivalent cation source added relative to 100 parts by mass of the uncolored silicate-coated body may be, for example, 12 parts by mass or less, 10 parts by mass or less, or 8 parts by mass or less.

Next, when the silicate-coated body is colored to a desired degree, the colored silicate-coated body is separated from the aqueous solvent by filtration or the like (S22; third separating step). Next, the separated colored silicate-coated body is dried (S23; third drying step). A colored silicate-coated body can be obtained thereby. In cases where the colored silicate-coated body does not need to be isolated, the separating step and the drying step do not have to be performed.

Typically, a substrate (e.g., mica) itself cannot be colored only by mixing the substrate and the ionic organic colorant. However, according to the manufacturing method of the sixth embodiment, even a substrate which is difficult to color directly can be colored (i.e., can be provided with a color). The substrate can be colored by a simple method.

According to the manufacturing method of the sixth embodiment, the substrate can be colored regardless of whether the ionic organic colorant is anionic, cationic or amphoteric. The substrate can be colored a desired color by selection and combination of the ionic organic colorants. Particularly, the substrate can be colored a color which the substrate alone cannot usually have.

A silicate-coated body according to a seventh embodiment of the present disclosure will be described.

The silicate-coated body according to the seventh embodiment further contains, in a silicate-coated body according to one of the first, second and fifth embodiments, an intermediate layer that coats at least part of the substrate. Reference can be made to the description above in the present disclosure regarding the silicate-coated bodies according to the first, second and fifth embodiments, and explanation thereon will be omitted hereinbelow.

The intermediate layer is preferably a material that can adhere physically and/or chemically to the substrate. The intermediate layer is preferably capable of tolerating the various production conditions for producing the first silicate. The intermediate layer is preferably a material to which the below-mentioned adhesive agent is physically and/or chemically adherable. The intermediate layer preferably has a size that allows it to be placed in a reaction vessel for producing the first silicate.

An example of the intermediate layer may include, although not limited to, a metal oxide film. Examples of the intermediate layer may include, although not limited to, metal oxides capable of exhibiting interference colors on the substrate and metal oxides capable of coloring the substrate with pearl colors (pearly luster). For example, high-refractive-index metal oxides may preferably be used as metal oxides capable of exhibiting interference colors. For example, it is possible to select a metal oxide having a refractive index of 2 or greater, preferably 2.4 or greater, more preferably 2.6 or greater. More specifically, examples of the metal oxide film may include, although not limited to, titanium dioxide films and iron oxide films. The crystal structure of titanium dioxide may be rutile or anatase.

The intermediate layer may coat the substrate partially, or may coat the entire substrate. The percentage by which to coat the substrate with the intermediate layer can be suitably set depending on the use of the silicate-coated body. For example, in cases where an interference color is to be created by the intermediate layer, it is preferred to coat the entire substrate with the intermediate layer.

The thickness of the intermediate layer on the substrate may be varied to achieve desired reflected colors. For example, in cases where the intermediate layer is a rutile titanium dioxide, a thickness from 50 to 70 nm can give a silver color. A thickness from 80 to 100 nm can give a yellow color. A thickness from 105 to 125 nm can give a red color. A thickness from 135 to 155 nm can give a blue color. A thickness from 160 to 180 nm can give a green color.

In the silicate-coated body according to the seventh embodiment, the first silicate coats at least part of the intermediate layer. In cases where the intermediate layer coats the entire substrate, the first silicate will coat the substrate via the intermediate layer.

The silicate-coated body according to the seventh embodiment can achieve the same effects as those of the foregoing embodiments. Further, according to the seventh embodiment, providing, for example, an intermediate layer having a different appearance from that of the substrate can yield a silicate-coated body having a different appearance from that of the foregoing embodiments. Further, providing an intermediate layer having different properties (e.g., chemical and/or physical properties) from those of the substrate can achieve different properties (e.g., chemical and/or physical properties) from those of the foregoing embodiments, and/or can protect the substrate from external actions.

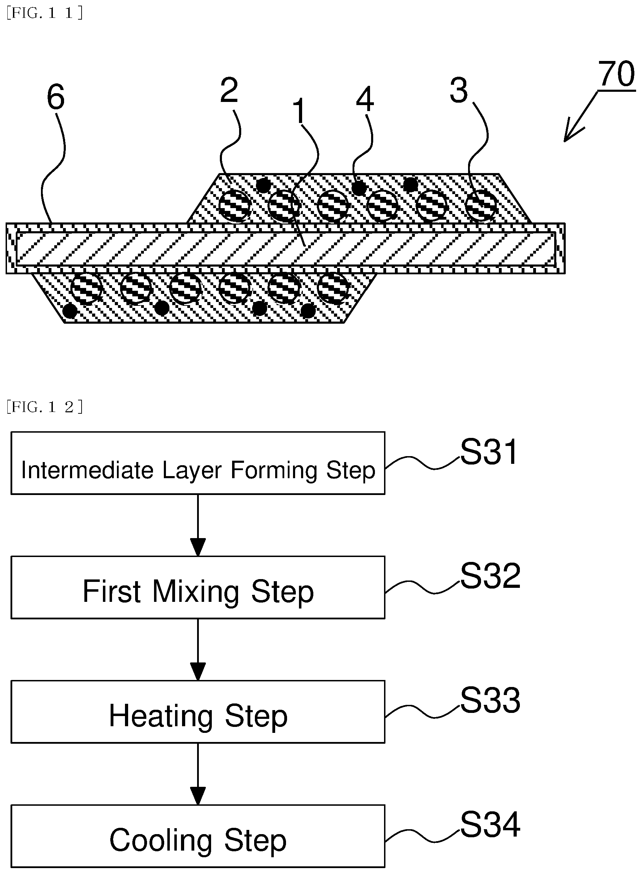

Silicate-coated bodies according to an eighth embodiment of the present disclosure will be described. FIGS. 10 and 11 show schematic sectional views of silicate-coated bodies according to the eighth embodiment. The silicate-coated body according to the eighth embodiment may be a combination of the second and seventh embodiments.

Silicate-coated bodies 60, 70 of the present disclosure further contain an adhesive agent 3, in addition to the substrate 1, the intermediate layer 6, the first silicate 2 and the functional substance 4 in the seventh embodiment. FIGS. 10 and 11 show configurations wherein the intermediate layer 6 covers the entire surface of the substrate 1, but the intermediate layer may coat only a portion of the substrate 1, as described above. The adhesive agent 3 may be present on the intermediate layer 6 and/or the substrate 1. At least a portion of the first silicate 2 may coat the intermediate layer 6 and/or substrate 1 via the adhesive agent 3. Configurations of the present embodiment, except that it contains the adhesive agent 3, are substantially the same as in the foregoing embodiments; therefore, reference can be made to the above description, and explanation thereon will be omitted hereinbelow.

The silicate-coated bodies according to the eighth embodiment can achieve the same effects as those of the silicate-coated bodies according to the foregoing embodiments. The presence of the adhesive agent can enhance the joining ability between the first silicate and the intermediate layer and/or substrate.

Silicate-coated bodies according to a ninth embodiment of the present disclosure will be described. The ninth embodiment may be a combination of the fifth embodiment and the seventh and/or eighth embodiment(s).

Silicate-coated bodies according to the ninth embodiment further contain an ionic organic colorant, in addition to the configurations of the seventh and eighth embodiments. The configuration of the ionic organic colorant, as well as its relationship with the first silicate, is substantially the same as in the foregoing embodiments; therefore, reference can be made to the above description, and explanation thereon will be omitted hereinbelow. Also, the multivalent cations to be included in cases where the ionic organic colorant is an anionic organic colorant are substantially the same as in the foregoing embodiments; therefore, reference can be made to the above description, and explanation thereon will be omitted hereinbelow.

The ninth embodiment can achieve the same effects as those of the fifth embodiment. Particularly, providing an intermediate layer having an interference color or pearl color can yield a silicate-coated body having an appearance in which the color of the intermediate layer is combined with the color of the ionic organic colorant.

As a tenth embodiment of the present disclosure, a method for manufacturing the silicate-coated bodies according to the seventh to ninth embodiments will be described. FIG. 12 shows a flowchart of a manufacturing method according to the tenth embodiment. In the third embodiment, the first silicate is coated on the substrate. In contrast, in the seventh to ninth embodiments, a first silicate will be coated on a substrate having been coated with an intermediate layer.

The manufacturing method according to the tenth embodiment further involves a step S31 for coating the substrate with an intermediate layer (intermediate layer forming step) before the first mixing step (S11) of the third embodiment. Stated differently, in the first mixing step (S32), an intermediate-layer-coated substrate is used instead of the aforementioned substrate. The other steps in the tenth embodiment may be substantially the same as in the foregoing embodiments; therefore, reference can be made to the above description, and explanation thereon will be omitted hereinbelow. To manufacture silicate-coated bodies according to any of the foregoing embodiments, the various steps according to the foregoing embodiments may be performed after the cooling step (S34).

Any method may be employed for coating the substrate with the intermediate layer in the intermediate layer forming step (S31). For example, the method for coating the substrate with the intermediate layer may be a physical method or a chemical method. For example, in cases where the intermediate layer is a metal oxide film, any known method can be employed for coating the substrate with a metal oxide.

For example, in cases where the substrate is mica and titanium dioxide is to be coated on mica as the intermediate layer, first, titanium tetrachloride is hydrolyzed in a state where mica is dispersed in water, to thereby cause a precursor compound of titanium dioxide to deposit on mica. Then, the isolated product is fired, to thereby yield mica coated with titanium dioxide.

For example, in cases where the substrate is mica and iron oxide ($Fe_2O_3$ or $Fe_3O_4$) is to be coated on mica as the intermediate layer, iron oxide ($Fe_3O_4$) can be deposited on mica by oxidizing divalent iron ions ($Fe^{2+}$) in a state where mica is dispersed in water. Further, optionally firing the isolated product can yield mica coated with iron oxide ($Fe_2O3$).

The manufacturing method of the tenth embodiment can produce the silicate-coated bodies according to the seventh to ninth embodiments.

There may be cases where it is difficult, or utterly impractical, to directly define the silicate-coated body of the present disclosure based on the composition, structure, and/or properties thereof. In such circumstances, it should be permissible to define the silicate-coated body of the present disclosure according to methods for manufacturing the same.

Examples

Silicate-coated bodies and methods for manufacturing the same of the present disclosure will be described hereinafter by way of examples. The silicate-coated bodies and the methods for manufacturing the same are not, however, limited to the following examples.

Test Examples 1 to 6:

[Production of Hectorite-Coated Mica]

A silicate-coated mica having mica as a substrate and hectorite as a first silicate was produced. Synthetic mica (phlogopite; $KMg_3AlSi_3O_{10}F_2$), silica sol, LiF, $MgCl_2$, and urea were placed into water, and these were dispersed ultrasonically. Synthetic mica having a median particle size of 12 $\mu$m and an average thickness of 0.3 $\mu$m was used. Silica sol having an average particle size of 10 nm was used. Silica particles were spherical, non-porous, and hydrophilic. The mixing ratio of silica (in pure content) relative to 1 g of synthetic mica was varied as follows: 0.1 g (Test Example 1), 0.2 g (Test Example 2), 0.3 g (Test Example 3), 0.4 g (Test Example 4), 0.5 g (Test Example 5), and 1 g (Test Example 6). The mixing ratio among silica sol, LiF, $MgCl_2$ and urea, in terms of molar ratio among Si element, Li element, Mg element and urea, was 40:7:28:255. Next, the mixed liquid was subjected to heating and pressurizing at 100°C for 48 hours with an autoclave. Next, the reaction product was cooled rapidly, the solid content was then separated by centrifugal separation, and the separated solid content was dried. The obtained solid content was analyzed.

[X-Ray Diffraction Measurement]

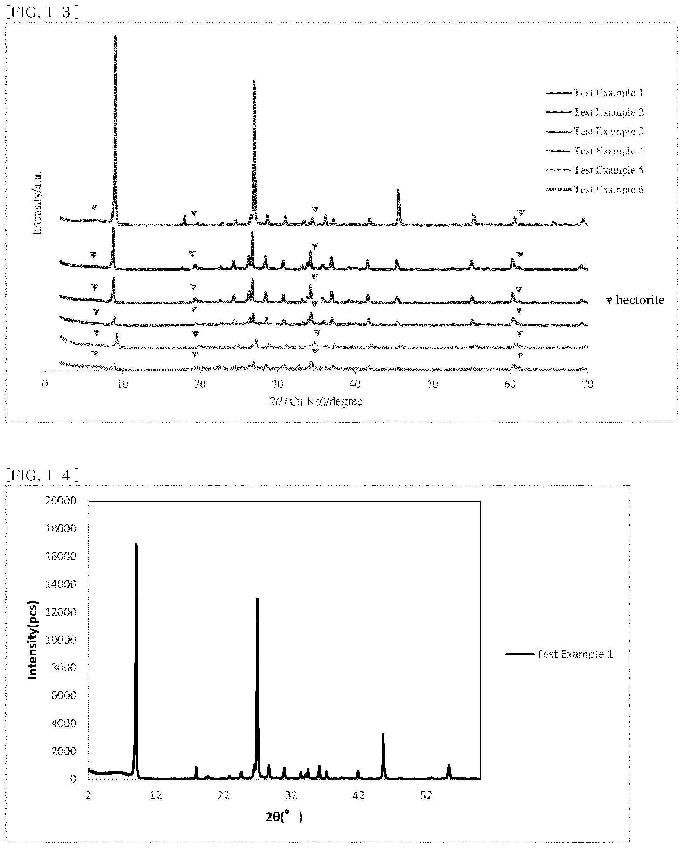

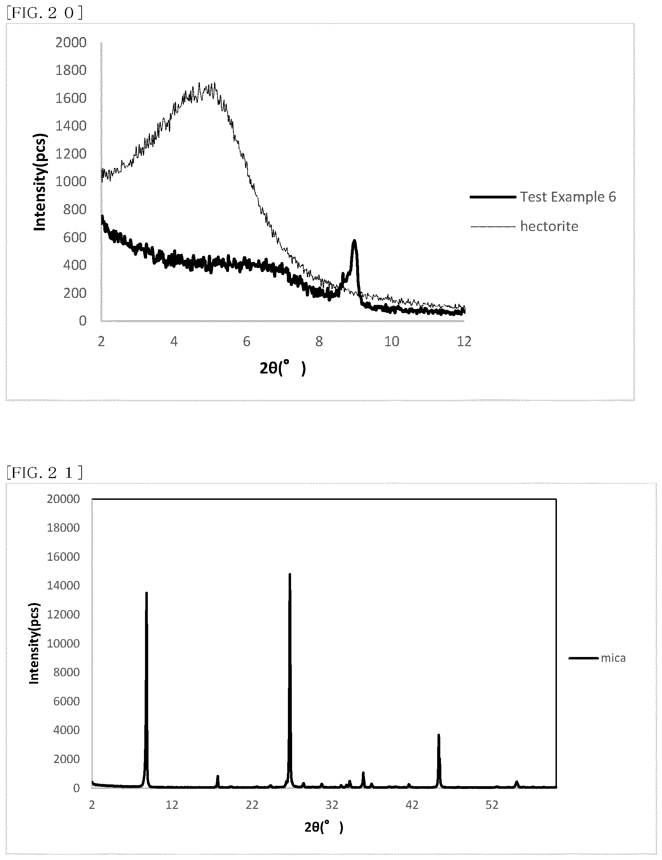

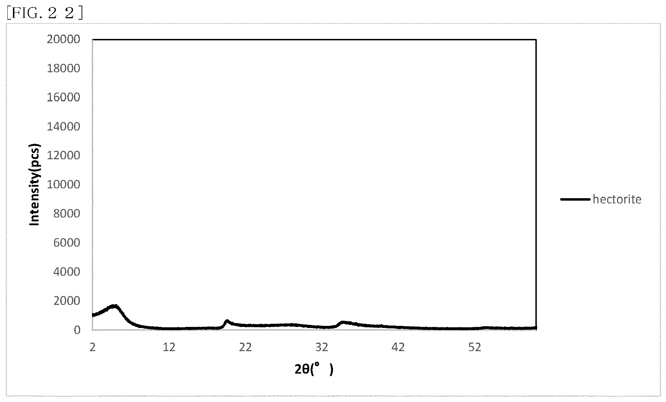

The reaction products of Test Examples 1 to 6 were subjected to X-ray diffraction measurement (CuKα rays; Rigaku RINT 2200 V/PC). FIG. 13 shows X-ray diffraction patterns of the reaction products obtained in Test Examples 1 to 6. The patterns shown in FIG. 13 show the patterns of Test Examples 1 to 6 in sequential order from the top. FIG. 21 shows an X-ray diffraction pattern of synthetic mica alone as a comparative control. FIG. 22 shows an X-ray diffraction pattern of hectorite alone. The peaks of hectorite appear in all the X-ray diffraction patterns shown in FIG. 13. This suggests that hectorite was produced in Test Examples 1 to 6. In the pattern of mica shown in FIG. 21, peaks appear, for example, at positions $2\theta$ = approx. 9°, 27°, and 45°. On the other hand, in the patterns shown in FIG. 13, peaks also appear at the same positions. This shows that mica remains in the products.

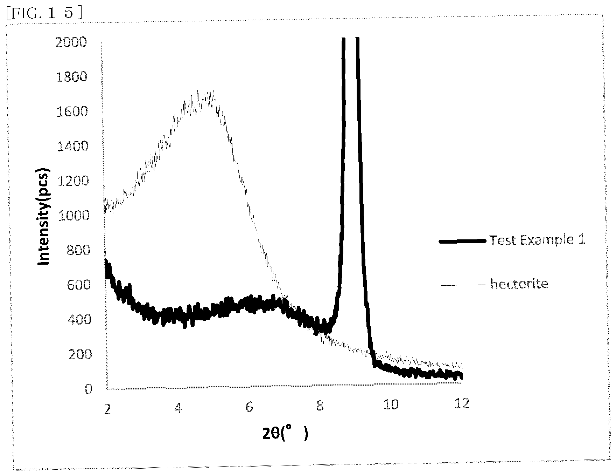

It is examined in more detail whether hectorite is produced or not by comparing the X-ray diffraction patterns of the reaction products with the X-ray diffraction pattern of hectorite. FIG. 14 shows the X-ray diffraction pattern of the reaction product according to Test Example 1. FIG. 15 shows the X-ray diffraction patterns of the product and hectorite, wherein the range of $2\theta$ = 2° to 12° in the patterns shown in FIG. 14 and FIG. 22 is shown enlarged. In the pattern of hectorite shown in FIG. 22, a broad peak exists in the range of $2\theta$ = 2° to 8°. On the other hand, in the pattern of mica shown in FIG. 21, no peak exists in the range of $2\theta$ = 2° to 8°. So, whether hectorite is produced or not can be determined by focusing on the peak of a reaction product in the range of $2\theta$ = 2° to 8°.

Referring to the pattern according to Test Example 1 shown in FIG. 15, a broad weak peak exists in the range of 4° to 8° as in the pattern of hectorite. It is thought that this peak is ascribable to hectorite. This thus suggests that the reaction product contains hectorite. In FIG. 15, the peak of Test Example 1 exists on the higher angle side than the peak of hectorite. This is thought to be because water entered between the layers of hectorite, and thereby the peak of Test Example 1 shifted to the higher angle side.

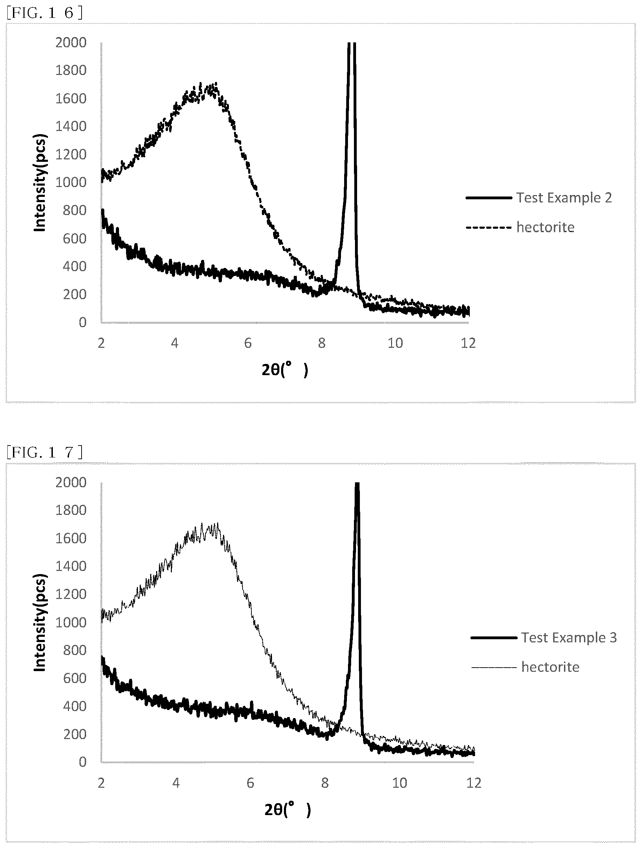

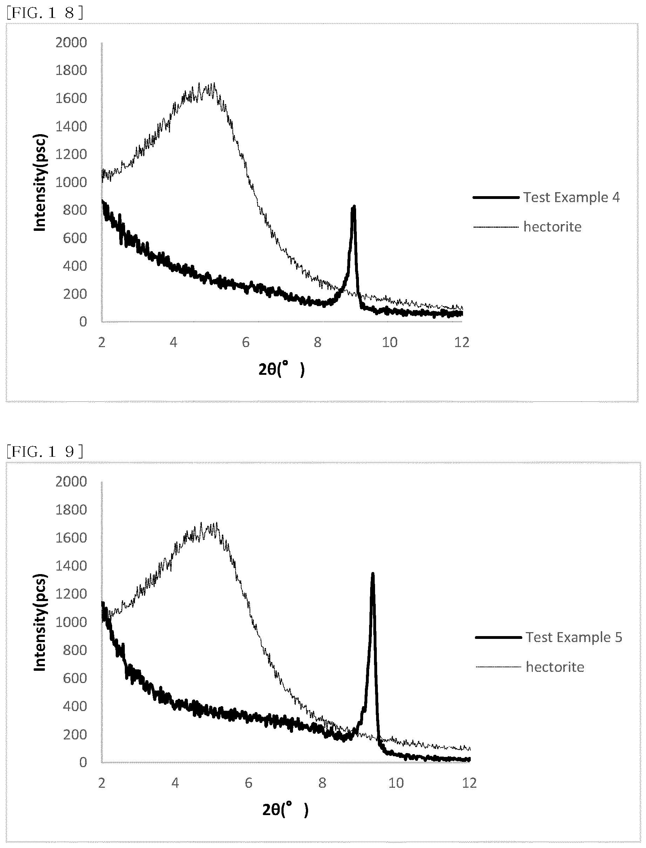

FIG. 16 to FIG. 20 respectively show the X-ray diffraction patterns of the products of Test Examples 2 to 6 and hectorite in the range of $2\theta$ = 2° to 12° as in FIG. 15. These X-ray diffraction patterns suggest that hectorite is produced also in Test Examples 2 to 6. Test Examples 4 to 6, however, show that an increase in the addition amount of silica tends to weaken the peak intensity of hectorite. This therefore suggests that the production amount of hectorite decreases with an increase in the addition amount of silica.

[Scanning Electron Microscope (SEM) Observation]

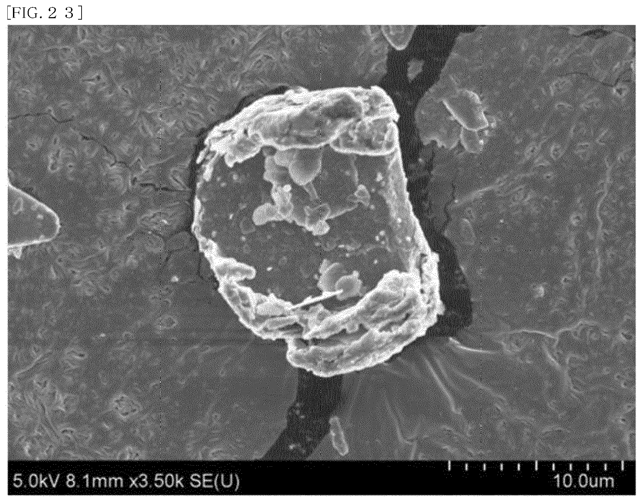

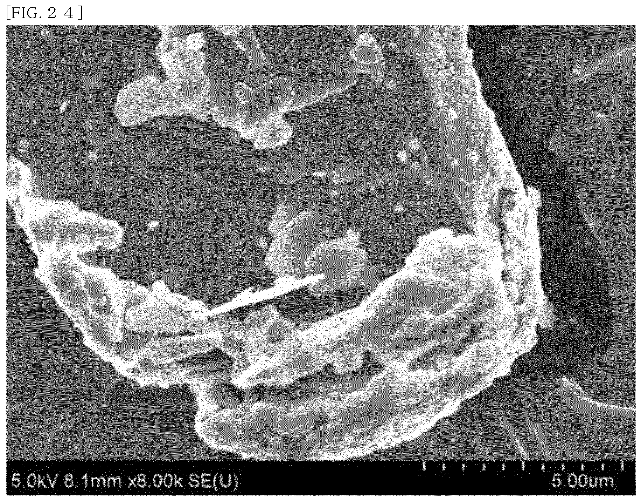

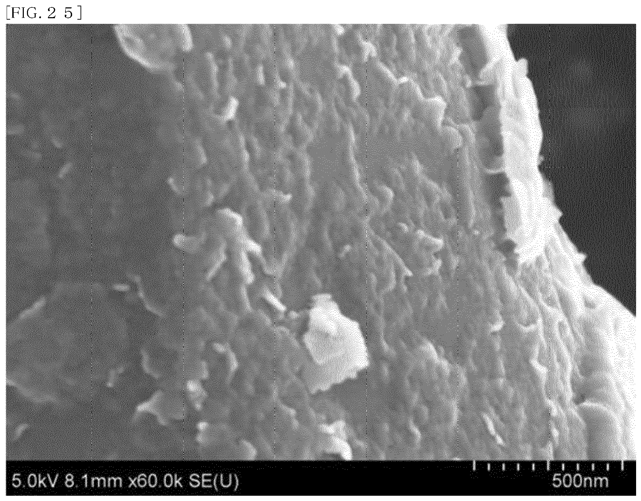

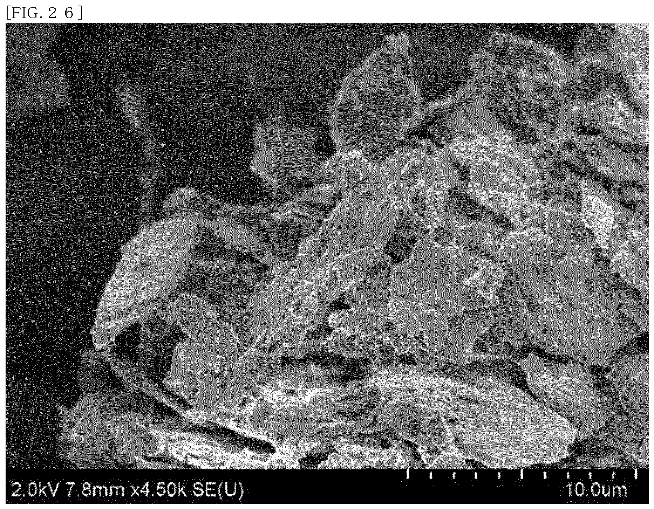

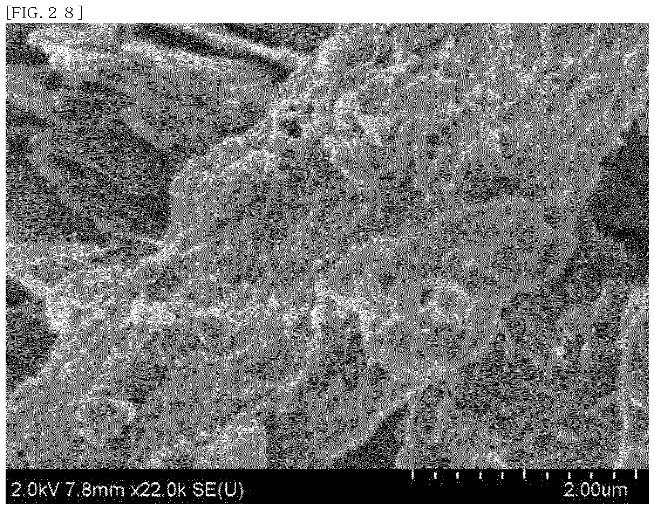

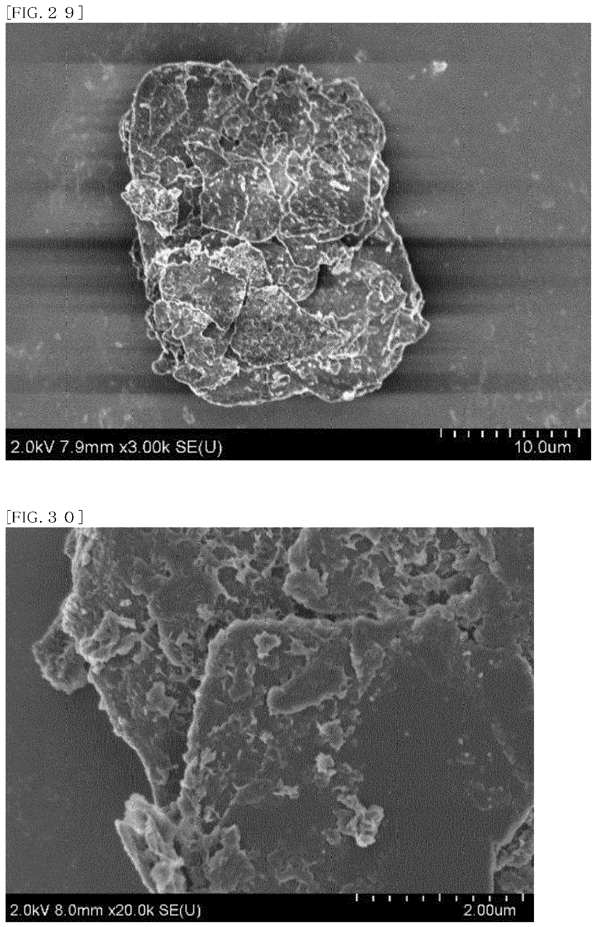

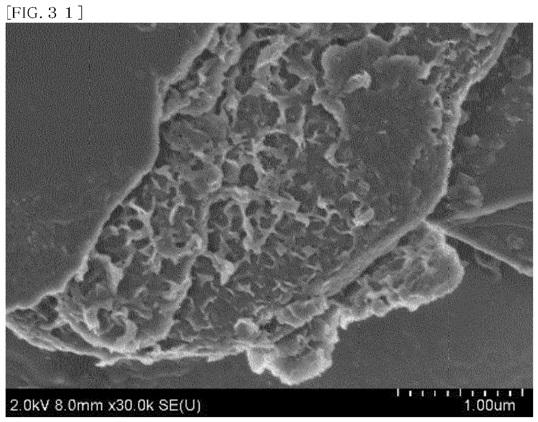

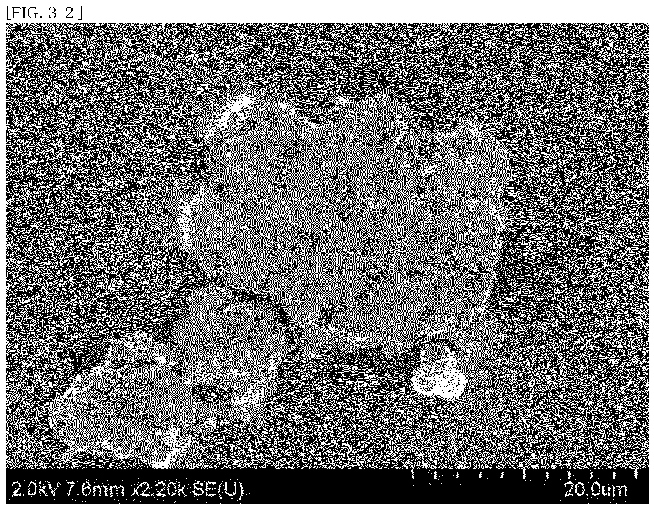

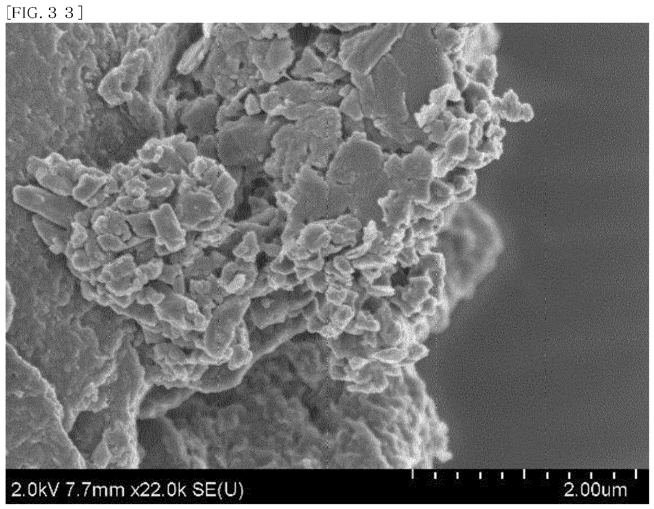

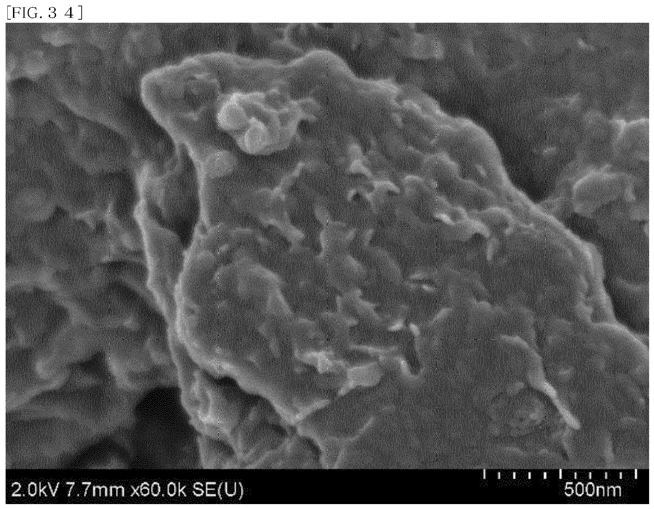

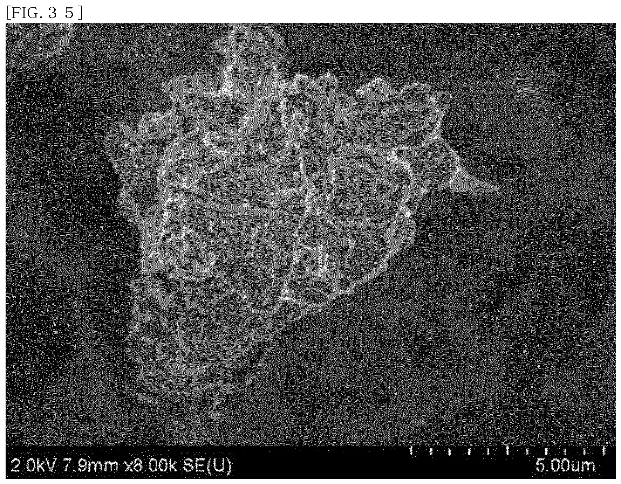

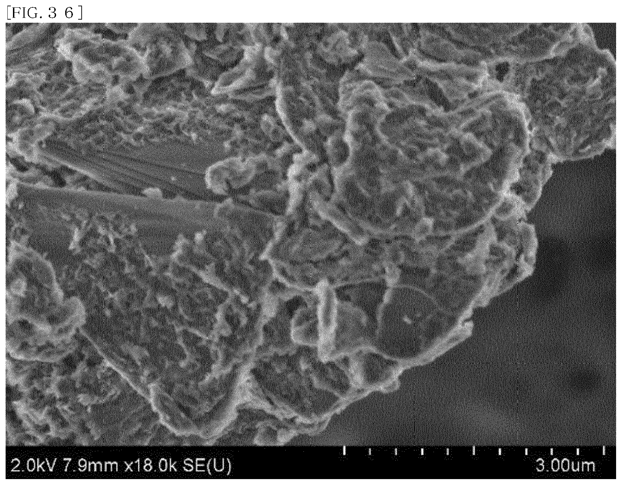

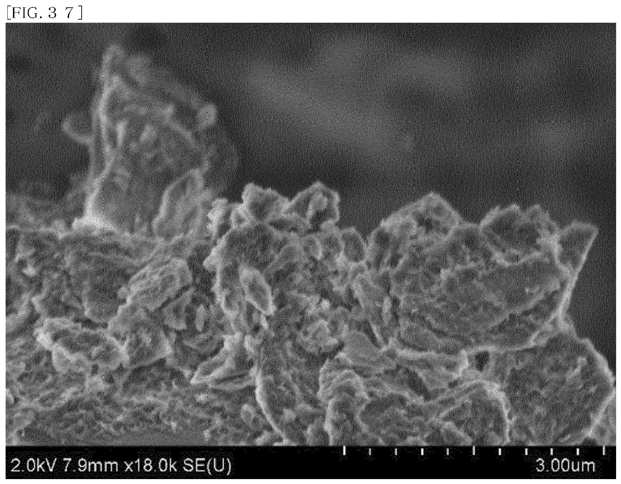

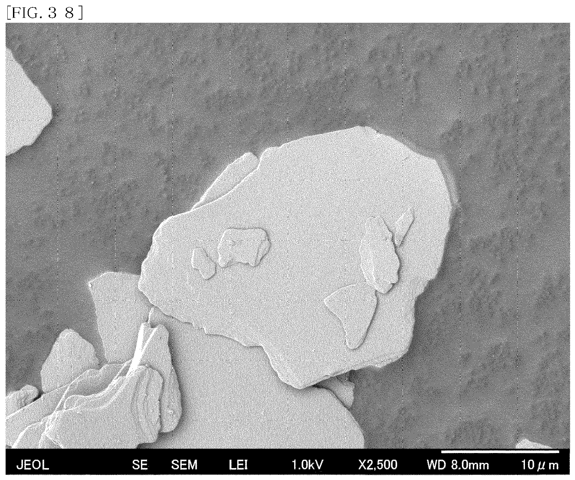

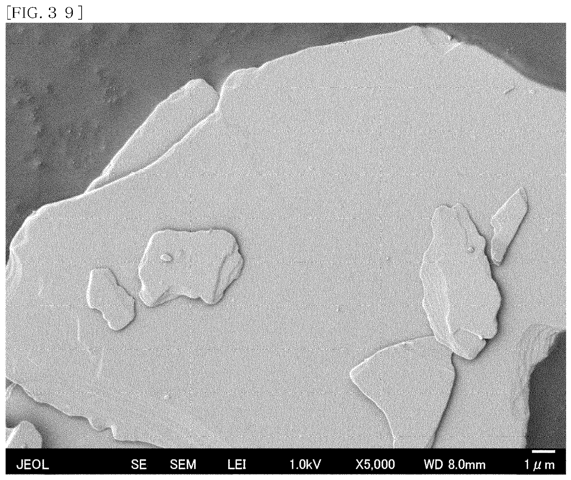

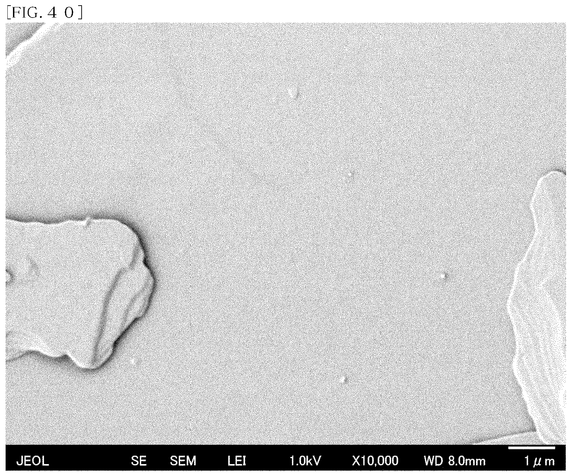

With respect to the products in Test Examples 1 to 5, the appearance and the surfaces of particles were observed using a field emission scanning electron microscope (Hitachi SU-8000). FIG. 23 to FIG. 25 show SEM images of the product in Test Example 1. FIG. 26 to FIG. 28 show SEM images of the product in Test Example 2. FIG. 29 to FIG. 31 show SEM images of the product in Test Example 3. FIG. 32 to FIG. 34 show SEM images of the product in Test Example 4. FIG. 35 to FIG. 37 show SEM images of the product in Test Example 5. FIG. 38 to FIG. 40 show SEM images of synthetic mica alone as comparative controls.

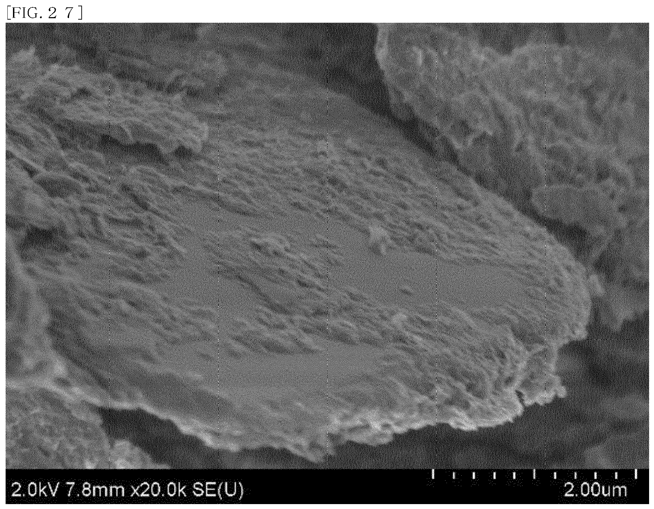

In the images of FIG. 23 to FIG. 37, platy particles (substrate particles) are mica particles. According to SEM images of mica alone shown in FIG. 38 to FIG. 40, mica has a smooth surface. On the other hand, the surfaces of the particles shown in FIG. 23 to FIG. 37 are not smooth (e.g., minute asperities are present). This therefore suggests that substances existing in unsmooth regions on the surface of the particles (fibrous projections or extraneous matters) are hectorite and/or silica in the images of FIG. 23 to FIG. 37. This thus suggests that all of Test Examples 1 to 5 were able to produce hectorite-coated mica. It is thought that regions which look smooth are portions in which mica is exposed, for example, in particles shown in FIG. 27. Meanwhile, aggregation and solidification of mica particles were observed as the addition amount of silica increased.

[Creation of Adsorption Isotherm]

Whether colorant deposition occurs on the respective reaction products of Test Examples 1 to 5 was verified by making the products adsorb methylene blue. Also, the production amount of hectorite was measured from the adsorption amount of methylene blue.

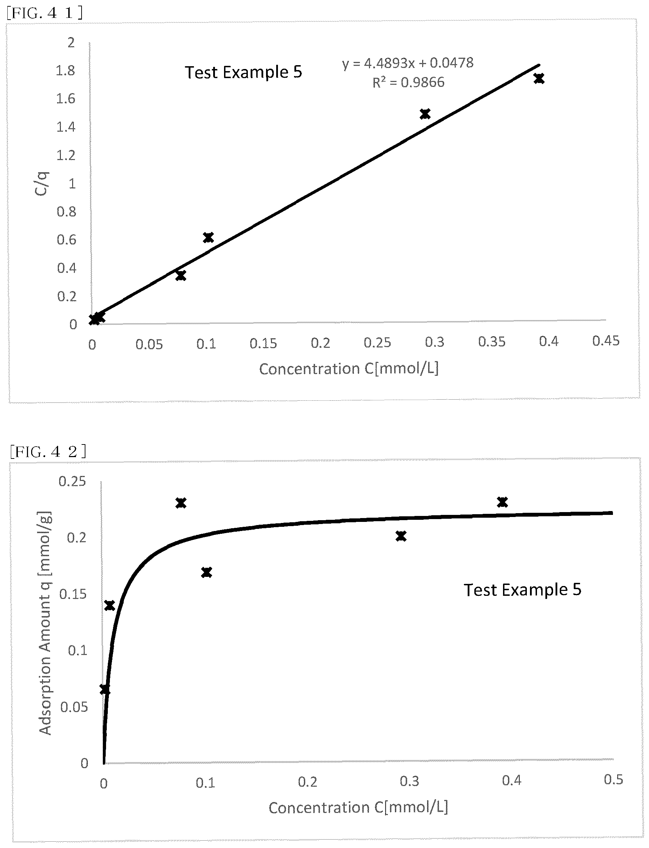

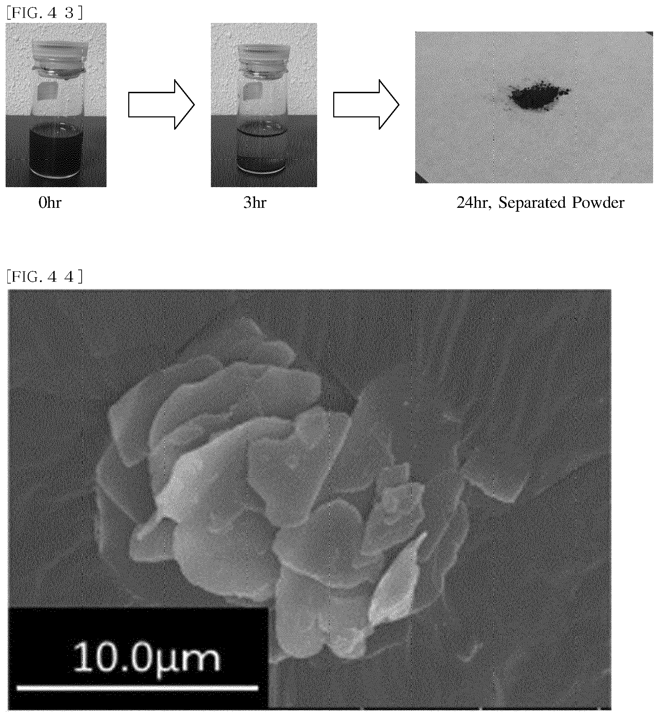

Six aqueous methylene blue solutions having different concentrations of methylene blue were prepared. Then, 50 mg of each product according to the respective Test Examples was immersed in 25 mL of each of the aqueous methylene blue solutions having the respective concentrations, and each aqueous solution was subjected to reciprocatory shaking at 25°C for 24 hours. Each shaken aqueous solution was subjected to centrifugal separation treatment (at 3 krpm for 10 minutes), and the absorbance ($\lambda$ = 665 nm) of the supernatant liquid was measured. The adsorption amount q (mmol/g) of methylene blue relative to 1 g of the product was calculated from the obtained absorbance. The measured values were plotted, with the horizontal axis indicating the methylene blue concentration (equilibrium concentration) C (mmol/L) and the vertical axis indicating the ratio, C/q (g/L), of the methylene blue concentration to the methylene blue adsorption amount, to find an approximate straight line. The saturated (maximum) adsorption amount $q_m$ (mmol/g) of methylene blue was found from the reciprocal of the slope of the approximate straight line, and the equilibrium coefficient K (L/mol) was found from the intercept. A theoretical curve of the adsorption isotherm was found from the obtained maximum adsorption amount $q_m$ and the equilibrium coefficient K. Table 1 shows the concentrations C of the prepared aqueous methylene blue solutions. Table 2 shows the calculated saturated adsorption amount $q_m$, the equilibrium coefficient K, and the correlation coefficient of the approximate straight line. For example, FIG. 41 and FIG. 42 respectively show an approximate straight line and a theoretical curve of the adsorption isotherm according to Test Example 5. FIG. 43 shows a photograph showing a state immediately after immersing a product in an aqueous methylene blue solution, a photograph showing a state of the solution after 3 hours from immersion, and a photograph showing a product separated from the aqueous solution 24 hours later, then washed and dried.

As shown in FIG. 43, the product was colored blue by the addition to the aqueous methylene blue solution. Hectorite adsorbs methylene blue, whereas mica and silica do not adsorb methylene blue. Therefore, the results suggest that hectorite has been formed in the products of Test Examples 1 to 5. Also, no unevenness in color was observed on the separated powder. This suggests that hectorite adhered to mica powder uniformly.

The addition amount of silica was increased in the order of Test Examples 1 to 5. Table 2 shows that there was an increase in the saturated adsorption amount $q_m$ from Test Example 1 to Test Example 2, but in Test Examples 2 to 5, there was no increase in the saturated adsorption amount $q_m$. This suggests that the production amount of hectorite depends on the addition amount of silica.

[Table 1]

| | Methylene Blue Concentration C(mmol/L) |
|---|---|
| Test Example 1 | 0.065, 0.130, 0.195, 0.26, 0.325, 0.390 |
| Test Examples 2-5 | 0.14, 0.28, 0.42, 0.56, 0.7, 0.84 |

[Table 2]

| | Saturated Adsorption Amount $q_m$ (mmol/g) | Equilibrium Coefficient K ($10^5$L/mol) | Correlation Coefficient $r^2$ |
|---|---|---|---|
| Test Example 1 | 0.11 | 8.4 | 0.99 |
| Test Example 2 | 0.24 | 0.34 | 0.95 |
| Test Example 3 | 0.24 | 0.26 | 0.95 |
| Test Example 4 | 0.10 | - | - |
| Test Example 5 | 0.22 | 0.94 | 0.99 |

The combined results of the X-ray diffraction measurement, SEM image analysis, and visible/ultraviolet spectroscopic analysis suggest that Test Examples 1 to 5 were able to produce hectorite-coated mica in which at least part of the mica particle was coated with hectorite. However, it is thought that, when the addition amount of silica is increased, silica acts as an adhesive and aggregates mica particles, and also, silica polymers are produced and thus cover the surface of mica, thereby inhibiting hectorite production and coating with hectorite. Therefore, it is thought that one of the conditions for making hectorite suitably adhere to mica is to set the content of silica to preferably 0.1 g or greater, more preferably 0.15 g or greater, relative to 1 g of mica. It is also thought that, to suppress the aggregation and solidification of the powder, it is preferred to set the content of silica to preferably 0.3 g or less, more preferably 0.25 g or less, relative to 1 g of mica.

Test Example 7:

To determine the relationship among hectorite, mica, and silica in the products obtained in Test Examples 1 to 6, a test was performed to verify whether hectorite adheres directly to mica. More specifically, a test was performed, as in Test Examples 1 to 6, using a mixture that contains synthetic mica, hectorite and urea, but does not contain any silica, magnesium compound or lithium compound. That is, synthetic mica, hectorite and urea were dispersed in water and subjected to heating and pressurizing treatment under the same conditions as in Test Examples 1 to 6, and then the treated substances were collected.

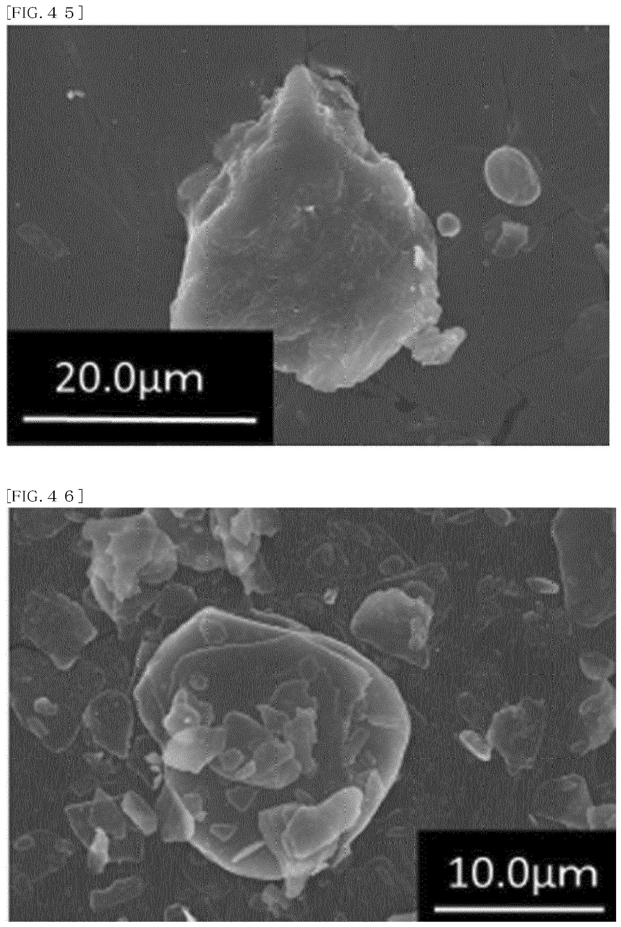

SEM images of the obtained treated substances were taken. FIG. 44 and FIG. 45 show SEM images of the treated substances. According to the SEM images, mica and hectorite existed separately, and no adhesion between mica and hectorite was observed. On the other hand, aggregation of hectorite was observed. This test thus suggests that, also in Test Examples 1 to 6, most of the produced hectorite does not adhere directly to mica.

Test Example 8:

To determine the relationship among hectorite, mica, and silica in the products obtained in Test Examples 1 to 6, a test was performed to verify whether silica adheres directly to mica. More specifically, a test was performed, as in Test Examples 1 to 6, using a mixture that contains synthetic mica, silica and urea, but does not contain any magnesium compound or lithium compound. That is, synthetic mica, silica sol and urea were dispersed in water and subjected to heating and pressurizing treatment under the same conditions as in Test Examples 1 to 6, and then the treated substances were collected.

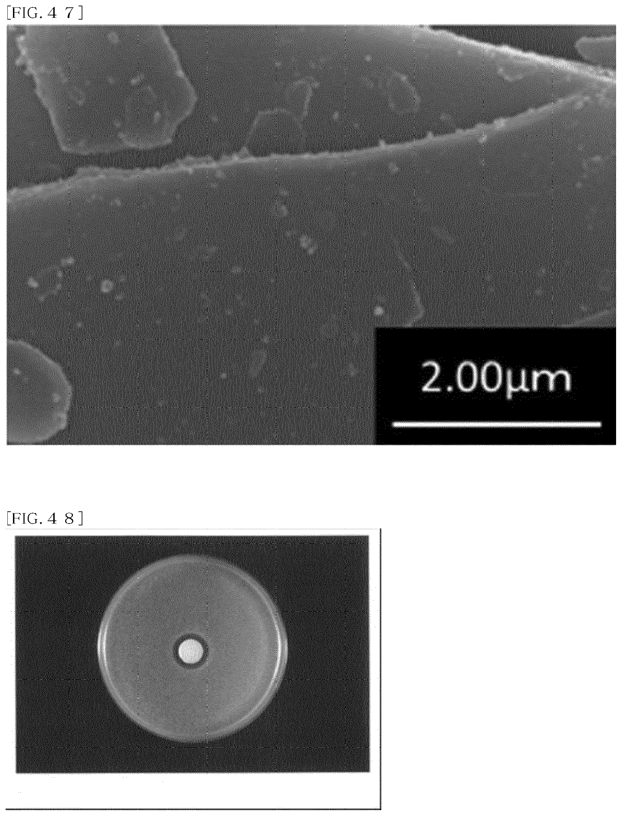

SEM images of the obtained treated substances were taken. FIG. 46 and FIG. 47 show SEM images of the treated substances. These SEM images show that silica particles adhered to the surface of mica. This test thus suggests that silica adheres to mica also in the products obtained in Test Examples 1 to 6.

According to Test Example 7, hectorite is not adsorbed on mica. According to Test Example 8, silica is adsorbed on mica. According to Patent Literature 2 and Non-Patent Literature 1, hectorite is produced on silica. These facts suggest that, in the hectorite coating process: first, silica (and/or a silicon compound derived from silica) adheres to mica; then, portions of the surface of adhered silica are attacked by hydroxide ions; and the attacked portions then react with other raw materials to thereby form hectorite, with silica serving as the starting point, thus resulting in that mica is coated by hectorite.

Therefore, it is thought that, according to the method of the present disclosure, it is possible to coat a substrate with a smectite-group silicate such as hectorite, even if the substrate has low hectorite adherability, so long as the substrate has silica adherability.

Test Examples 9 to 13:

An antibacterial substance (antibacterial agent) was carried as a functional substance on the silicate-coated body produced in Test Example 1. The antibacterial agents used were: diamminesilver chloride (Test Example 9), silver nitrate (Test Example 10), and silver acetate (Test Example 11).

In Test Example 9, first, 0.1 g of silver chloride, 10 g of water, and 0.2 g of 25% ammonia water were mixed, to prepare a diamminesilver chloride aqueous solution. Further, 30 g of the silicate-coated body produced in Test Example 1 was mixed with 270 g of water. The diamminesilver chloride aqueous solution was added gradually to the dispersion liquid of the silicate-coated body, and the mixture was stirred and mixed for at least 30 minutes. Next, the powder in the dispersion liquid was isolated by dehydration/filtration, and was then dispersed again in water and washed. Next, the powder was isolate by dehydration/filtration, and was then dried overnight at 110°C.

In Test Example 10, first, 2.5 g of a 0.5 M silver nitrate solution was added to 300 g of water, and the mixture was stirred at room temperature for 10 minutes. Then, 15 g of the silicate-coated body produced in Test Example 1 was added to this silver nitrate aqueous solution, and the mixture was stirred at room temperature for 1.5 hours. Next, the powder was isolated by dehydration/filtration, and was then dried overnight at 110°C.

In Test Example 11, first, 0.2 g of a 97% silver acetate aqueous solution was added to 300 g of water, and the mixture was stirred at room temperature for 10 minutes. Then, 15 g of the silicate-coated body produced in Test Example 1 was added to this silver acetate aqueous solution, and the mixture was stirred at room temperature for 1.5 hours. Next, the powder was isolated by dehydration/filtration, and was then dried overnight at 110°C.

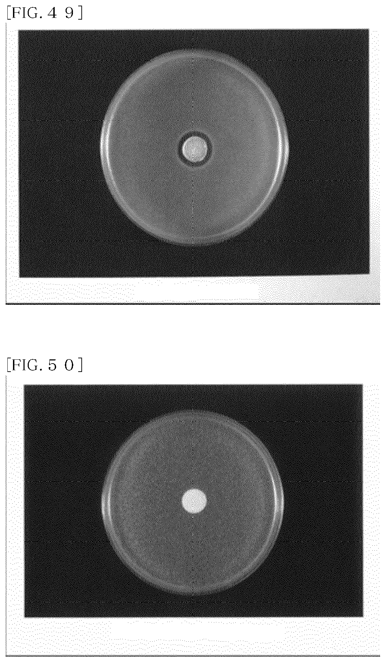

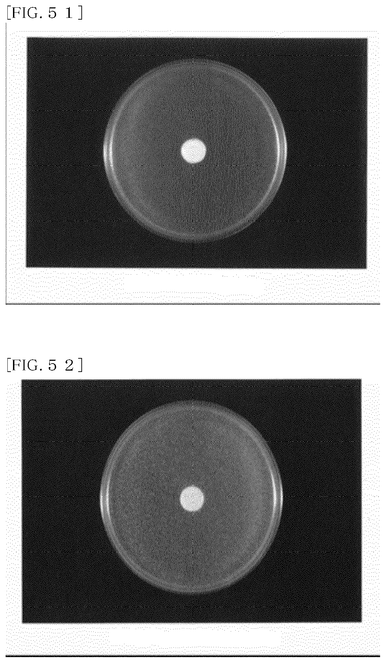

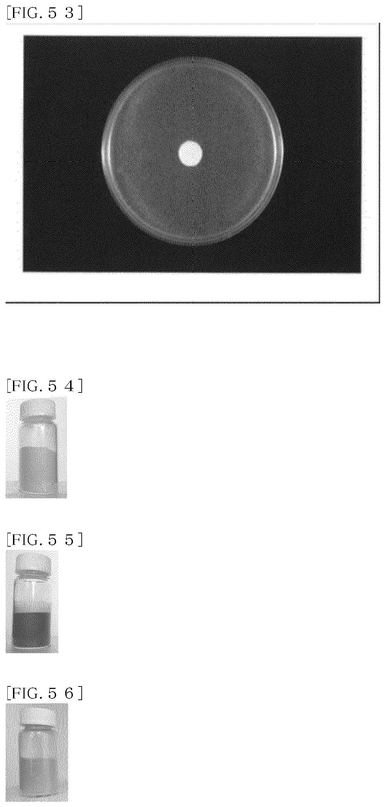

Tests were performed to verify whether the antibacterial agents were carried by the silicate-coated bodies in Test Examples 9 to 11, and whether the silicate-coated bodies exhibited antibacterial actions. The antibacterial tests of the silicate-coated bodies were performed with reference to the halo method described in JIS L 1902 (2015) titled "Determination of Antibacterial Activity and Efficacy of Textile Products." *Staphylococcus aureus* subsp. *aureus* (NBRC 12732) and *Escherichia coli* (NBRC 3301) were used as bacterial strains for the tests. As comparison controls, in Test Examples 12 and 13, antibacterial tests were performed respectively for synthetic phlogopite (with no silicate coating) and the silicate-coated body produced in Test Example 1 (with no antibacterial agent carried). Table 3 shows the conditions and results. The silicate-coated body concentration "4 g/10 g" in each pellet, as shown in Table 3, indicates that 4 g of the silicate-coated body is contained in 10 g of a mixture of the silicate-coated body and purified water. FIG. 48 shows a photograph of a halo test in Test Example 9-1. FIG. 49 shows a photograph of a halo test in Test Example 9-2. FIG. 50 shows a photograph of a halo test in Test Example 12-1. FIG. 51 shows a photograph of a halo test in Test Example 12-2. FIG. 52 shows a photograph of a halo test in Test Example 13-1. FIG. 53 shows a photograph of a halo test in Test Example 13-2.

FIG. 50 to FIG. 53 show that no "halo" (growth inhibition zone) was formed around the respective samples of synthetic mica in Test Example 12 and the non-antibacterial-agent-carrying silicate-coated body in Test Example 13. In contrast, FIG. 48 and FIG. 49 show that, in Test Examples 9 to 11, a halo was formed around each sample.

These results show that the first silicate in the silicate-coated body can be made to carry an ionic functional substance, such as an antibacterial substance, by ion exchange. The results suggest that, for example, in Test Examples 9 to 11, diamminesilver ions or silver ions are carried as antibacterial substances by the first silicate.

It was found that, by making the first silicate in the silicate-coated body carry a functional substance, functions ascribable to the functional substance can be exerted. For example, it was found that, by making the first silicate of the silicate-coated body carry an antibacterial substance, antibacterial properties can be imparted to the silicate-coated body.

[Table 3]

| | Sample | Antibacterial agent material | Concentration | Tested bacteria | Bacteria concentration (/ml) | Halo width (mm) |
|---|---|---|---|---|---|---|
| Test Example 9-1 Test Example 9-2 | Antibacterial-agent-carrying Silicate-coated Body | Diamminesilver Chloride | 4g/10g | *S. aureus* | $6.2 \times 10^6$ | 2.5 |
| | | | | *E. coli* | $9.5 \times 10^6$ | 3.0 |
| Test Example 10-1 Test Example 10-2 | Antibacterial-agent-carrying Silicate-coated Body | Silver Nitrate | 4g/10g | *S. aureus* | $5.0 \times 10^6$ | 1.6 |
| | | | | *E. coli* | $7.4 \times 10^6$ | 1.6 |
| Test Example 11-1 Test Example 11-2 | Antibacterial-agent-carrying Silicate-coated Body | Silver Acetate | 4g/10g | *S. aureus* | $5.0 \times 10^6$ | 1.3 |
| | | | | *E. coli* | $7.4 \times 10^6$ | 1.8 |
| Test Example 12-1 Test Example 12-2 | Synthetic Mica | - | 4g/10g | *S. aureus* | $4.7 \times 10^6$ | - |
| | | | | *E. coli* | $5.2 \times 10^6$ | - |
| Test Example 13-1 Test Example 13-2 | Non-carrying Silicate-coated Body | - | 4g/10g | *S. aureus* | $4.7 \times 10^6$ | - |
| | | | | *E. coli* | $5.2 \times 10^6$ | - |

Test Examples 14 to 19:

Colored silicate-coated bodies colored with ionic organic colorants were produced. The silicate-coated body (uncolored) produced in Test Example 1 was used as a raw material. As cationic organic colorants, methylene blue (Test Examples 14 and 15) and rhodamine B (Test Example 16) were used. As anionic organic colorants, brilliant blue FCF (Test Example 17), erythrosine B (Test Example 18), and tartrazine (Test Example 19) were used. The amounts of methylene blue were varied in Test Examples 14 and 15. The CIE 1976 L*a*b* color space (JIS Z8781) was measured for each obtained colored silicate-coated body. The color space was measured by filling a powder cell with 0.7 g of each sample and using a color difference meter CR-400 manufactured by Konica Minolta, Inc. Table 4 shows addition rates of the ionic organic colorants and the color tones of the colored silicate-coated bodies. The addition proportions shown in Table 4 indicate the proportions (parts by mass) of respective colorants added relative to 100 parts by mass of an uncolored silicate-coated body. The details of each Test Example will be described hereinafter.

[Table 4]

| | Ionic Organic Colorant | | Cation Source (Parts by Mass) | Color Tone | | |
|---|---|---|---|---|---|---|
| | Name | Parts by Mass | | L * | a * | b * |
| Test Example 14 | Methylene Blue | 0.05 | - | 82.95 | -9.18 | -10.95 |
| Test Example 15 | Methylene Blue | 0.90 | - | 54.23 | 3.61 | -20.24 |
| Test Example 16 | Rhodamine B | 0.27 | - | 74.62 | 36.81 | -13.82 |
| Test Example 17 | Brilliant blue FCF | 0.20 | 4 | 74.33 | -11.87 | -20.40 |
| Test Example 18 | Erythrosine B | 0.20 | 4 | 77.80 | 31.84 | -0.07 |
| Test Example 19 | Tartrazine | 0.40 | 6 | 90.70 | -3.37 | 33.15 |

In Test Examples 14 and 15, methylene blue was adsorbed by the uncolored silicate-coated body by the same method as the method in which the colored samples were produced to create the above adsorption isotherm in Test Examples 1 to 5. FIG. 54 and FIG. 55 show photographs of the colored silicate-coated bodies obtained in Test Examples 14 and 15, respectively. FIG. 54 shows that, in Test Example 14 in which the methylene blue concentration was low, it was possible to obtain a silicate-coated body colored light blue. FIG. 55 shows that, in Test Example 15 in which the methylene blue concentration was high, it was possible to obtain a silicate-coated body colored dark blue. Therefore, it was found that, by changing the adsorption amount of ionic organic colorant in accordance with the addition proportion of the ionic organic colorant, the darkness/lightness in the color tone of the colored silicate-coated body can be adjusted.

In Test Example 16, the uncolored silicate-coated body was added to water so that the concentration was 10% by mass. Next, rhodamine B was added at an addition proportion shown in Table 4, and the mixture was stirred for 1 hour. Next, the product was dehydrated and filtered by centrifugal separation, and then dried at 100°C. The dried product was passed through a 120-mesh sieve to obtain a colored silicate-coated body. FIG. 56 shows a photograph of the colored silicate-coated body obtained in Test Example 16. It was possible to obtain a silicate-coated body colored pink.

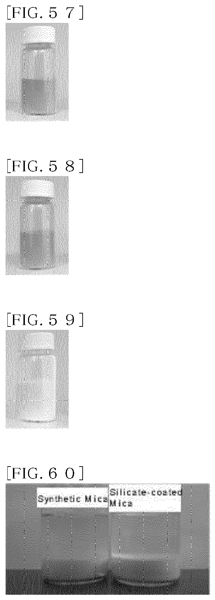

In each of Test Examples 17 to 19, the uncolored silicate-coated body was added to water so that the concentration was 10% by mass. Next, the respective organic colorant was added at an addition proportion shown in Table 4. Next, as a multivalent cation source, aluminum chloride hydrate was added at an addition proportion shown in Table 4, and the mixture was stirred for at least 1 hour. Next, the product was dehydrated and filtered by centrifugal separation, and then dried at 100°C. The dried product was passed through a 120-mesh sieve to obtain a colored silicate-coated body. FIG. 57 to FIG. 59 show photographs of the colored silicate-coated bodies obtained in Test Examples 17 to 19, respectively. In Test Example 17, it was possible to obtain a silicate-coated body colored blue. In Test Example 18, it was possible to obtain a silicate-coated body colored red. In Test Example 19, it was possible to obtain a silicate-coated body colored yellow.

As a comparative example of Test Example 19, a coloring step was performed by the same method as in Test Example 19 except that synthetic mica having the same particle size was used as a coloring target, instead of the uncolored silicate-coated body. FIG. 60 shows the mixed liquids in a state left standing before centrifugal separation/dehydration in the respective coloring processes of Test Example 19 and Comparative Example. In the mixed liquid using the silicate-coated body as a coloring target (on the right side), the supernatant liquid became transparent. In contrast, in the mixed liquid using synthetic mica as a coloring target (on the left side), the supernatant liquid remained cloudy. This shows that the colorant was not adsorbed on synthetic mica. Therefore, it is thought that the ionic organic colorant is adsorbed by hectorite.

Accordingly, it was found that the method of the present disclosure is useful for coloring mica. It was also found that a substrate, such as mica, colored a desired color can be obtained by using an ionic organic colorant.

Tests were performed to verify whether or not the colorants desorb from the colored silicate-coated bodies produced in Test Examples 14 to 19. Also, removability from the skin was compared for each of the colored silicate-coated bodies and the ionic organic colorants. Each of the colored silicate-coated bodies of Test Examples 14 to 19, as well as each of the ionic organic colorants, was applied to the skin and then washed lightly with water, to visually verify whether or not each color came off from the skin. The evaluation criteria for color removability are shown below. Table 5 shows the results.

A: Color can be removed from the skin only by washing with water lightly.
B: Color cannot be removed from the skin just by washing with water lightly.

In cases where an ionic organic colorant itself was applied to the skin, the color remained on the skin, because the colorant was embedded in the skin grooves even when washed with water. In contrast, as for the colored silicate-coated bodies, it was possible to remove the color from the skin easily only by washing with water. From these results, it was confirmed that an ionic organic colorant adsorbed to a colored silicate-coated body does not easily desorb from the silicate-coated body. It was also confirmed that the colored silicate-coated bodies are easily removable.

[Table 5]

| Applied Substance | Evaluation of color removability |
|---|---|
| Colored silicate-coated body of Test Example 14 | A |
| Colored silicate-coated body of Test Example 15 | A |
| Colored silicate-coated body of Test Example 16 | A |
| Colored silicate-coated body of Test Example 17 | A |
| Colored silicate-coated body of Test Example 18 | A |
| Colored silicate-coated body of Test Example 19 | A |
| Methylene Blue | B |
| Rhodamine B | B |
| Brilliant blue FCF | B |
| Erythrosine B | B |
| Tartrazine | B |

Test Examples 20 to 28:

Colored silicate-coated bodies colored with ionic organic colorants were produced. The method for adsorbing each ionic organic colorant is basically the same as the method employed in Test Examples 16 to 19. The uncolored silicate-coated body produced in Test Example 1 was added to water so that the concentration was 10% by mass. Table 6 shows the ionic organic colorants that were used and their respective addition proportions. The addition proportions shown in Table 6 indicate the proportions (parts by mass) of respective colorants added relative to 100 parts by mass of an uncolored silicate-coated body.

In Test Examples 20 to 23, 4 parts by mass of aluminum chloride hydrate was further added as a multivalent cation source, and the mixture was stirred. Next, 0.8 parts by mass of sodium hydroxide was added to promote the adsorption of the colorant ions, and the mixture was stirred for at least 1 hour. Then, 0.7 parts by mass of aluminum chloride hydrate was further added as a multivalent cation source, and the mixture was stirred for at least 1 hour.

In Test Examples 24 to 26, 4 parts by mass of aluminum chloride hydrate was further added as a multivalent cation source, and the mixture was stirred.

In Test Examples 27 and 28, 6 parts by mass of aluminum chloride hydrate was further added as a multivalent cation source, and the mixture was stirred. Next, 0.8 parts by mass of sodium hydroxide was added to promote the adsorption of the colorant ions, and the mixture was stirred for at least 1 hour. Then, 0.7 parts by mass of aluminum chloride hydrate was further added as a multivalent cation source, and the mixture was stirred for at least 1 hour.

In each of Test Examples 20 to 28, the product was then dehydrated and filtered by centrifugal separation, and then dried at 100°C. The dried product was passed through a 120-mesh sieve to obtain each colored silicate-coated body.

In all of Test Examples 20 to 28, it was possible to color the silicate-coated body with the respective colors of the colorants. Also, color formation was uniform and vivid. Further, like the colored silicate-coated bodies produced in Test Examples 14 to 19, the colored silicate-coated bodies produced in Test Examples 20 to 28 exhibited no color migration from each colored silicate-coated body to the surface in contact. Furthermore, these silicate-coated bodies did not undergo aggregation caused by adsorption of ionic organic colorants, and thus, powders of these colored silicate-coated bodies exhibited excellent texture to the touch.

[Table 6]

| | Ionic Organic Colorant | | Cation Source (Parts by Mass) | Alcali Source (Parts by Mass) |
|---|---|---|---|---|
| | Name | Parts by Mass | | |
| Test Example 20 | Amaranth | 0.1 | 4+0.7 | 0.8 |
| Test Example 21 | New Coccine | 0.2 | 4+0.7 | 0.8 |
| Test Example 22 | Phloxine B | 0.2 | 4+0.7 | 0.8 |
| Test Example 23 | Rose Bengal | 0.2 | 4+0.7 | 0.8 |
| Test Example 24 | Acid Red | 0.1 | 4 | - |
| Test Example 25 | Fast Green FCF | 0.2 | 4 | - |
| Test Example 26 | Indigo Carmine | 0.1 | 4 | - |
| Test Example 27 | Sunset Yellow FCF | 0.3 | 6+0.7 | 0.8 |
| Test Example 28 | Lithol Rubine B | 0.4 | 6+0.7 | 0.8 |

Test Example 29:

A colored silicate-coated body colored by a plurality of ionic organic colorants was produced. The silicate-coated body produced in Test Example 1 was used as a raw material. Methylene blue and rhodamine B were used as cationic organic colorants.

First, silicate-coated mica and water were mixed so that the solid-content concentration was 4% by mass. Next, 0.3 parts by mass of methylene blue was added relative to 100 parts by mass of the silicate-coated mica, and the mixture was stirred. Then, 0.2 parts by mass of rhodamine B was added relative to 100 parts by mass of the silicate-coated mica, and the mixture was stirred. After continuing stirring for at least 1 hour, water was separated by dehydration/filtration. The obtained powder was dried at 100°C and then passed through a 120-mesh sieve, to obtain a resultant product.

The resultant product was a purple powder, having both the colors of methylene blue and rhodamine B. This shows that, by combining a plurality of organic colorants, it is possible to color a silicate-coated body with a desired color.

Test Example 30:

A silicate-coated body was produced by coating a first silicate on an intermediate-layer-coated substrate. Synthetic mica was used as the substrate. The synthetic mica was coated with titanium dioxide as an intermediate layer. Hectorite was synthesized as the first silicate.

To 1 L of ion-exchanged water, 50 g of synthetic mica was dispersed while being stirred, and the dispersion liquid was heated to 75°C. The pH of this dispersion liquid was adjusted to 1.6 using 20% hydrochloric acid. Next, 15 g of a 10% tin (II) chloride aqueous solution including 3 g of hydrochloric acid was added to the dispersion liquid in 30 minutes, and during this time, the pH of the dispersion liquid was maintained at 1.6 using a 20% sodium hydroxide aqueous solution. After completing the addition, the dispersion liquid was further stirred for 20 minutes. Then, 110 g of a 40% titanium tetrachloride aqueous solution was added to the dispersion liquid at a rate of 30 g/h, while maintaining the pH at 1.6 using a 20% sodium hydroxide aqueous solution. Then, the dispersion liquid was neutralized to pH 7 using a 20% sodium hydroxide aqueous solution. Next, the dispersion liquid was dehydrated, and the product was washed, dried, and then fired at 800°C. As a result, an intermediate-layer-coated substrate was obtained, the substrate being coated with titanium dioxide and having a golden interference color (pearly luster).

Then, 1 g of the obtained intermediate-layer-coated substrate, starting materials of hectorite (i.e., 0.015 g of LiF, 0.47 g of MgCh, and 0.1 g of silica), and 0.64 g of urea were dispersed in water ultrasonically. The mixed liquid was placed in an autoclave and subjected to hydrothermal treatment (at 100°C for 48 h). After rapid cooling, a centrifugal separator (3000 rpm) was used to subject the mixed liquid to solid-liquid separation and washing three times. The separated solid content was dried with a dryer (at 50°C for 24 h), to obtain a silicate-coated body including an intermediate layer.

Coating with a first silicate was possible, even in cases where the substrate was coated with an intermediate layer consisting of a metal oxide film. Further, even when the substrate was covered with the first silicate, the powder's appearance exhibited an interference color (pearl color) ascribable to the intermediate layer.

Test Example 31:

A colored silicate-coated body was produced by coloring the intermediate-layer-equipped silicate-coated body produced in Test Example 30 with an ionic organic colorant. The coloring method was the same as that employed in Test Examples 16 to 28. More specifically, the silicate-coated body produced in Test Example 30 and water were mixed so that the solid-content concentration was 10%. Next, 0.27 parts by mass of rhodamine B relative to 100 parts by mass of the silicate-coated body was added to the mixture. The mixture was stirred for at least 1 hour, and was then subjected to dehydration/filtration, to isolate a powder. The obtained powder was dried at 100°C and then passed through a 120-mesh sieve, to obtain a colored silicate-coated body.

The obtained colored silicate-coated body was colored, like the colored silicate-coated bodies produced in the aforementioned Test Examples. This shows that coloring is possible even in cases where an intermediate layer is provided. This also shows that, by coloring a powder having a pearl color (interference color), it is possible to obtain a powder exhibiting, in combination, a pearl color (interference color) and the color of the ionic organic colorant.

The silicate-coated bodies and manufacturing methods therefor of the present disclosure have been described according to the foregoing embodiments and examples, but they are not limited to the foregoing embodiments and examples and may encompass various transformations, modifications, and improvements made to the various disclosed elements (including elements disclosed in the Claims, Description, and Drawings) within the scope of the invention and according to the fundamental technical idea of the invention. Further, various combinations, substitutions, and selections of the various disclosed elements are possible within the scope of the claims of the invention.

Further issues, objectives, and embodiments (including modifications) of the invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

Industrial Applicability

The silicate-coated body of the present disclosure is applicable, for example, to cosmetics, paint, metal ion adsorbents, films, nanocomposite materials, and the like.

For example, in cases where hectorite-coated mica powder is to be used as a nanocomposite material for a film (e.g., gas-barrier film), increasing the surface area of mica particles with hectorite can improve barrier properties as well as adhesiveness with the film, and can also improve mechanical properties such as tensile strength.

List of reference symbols

1 Substrate
2 Adhesive agent
3 First silicate
4 Functional substance
5 Ionic organic colorant
6 Intermediate layer
10, 20, 40, 50, 60, 70 Silicate-coated body
31 Sheet structure
32 Functional substance, Ionic organic colorant
33 Multivalent cation

**Claims**

1. A silicate-coated body comprising:

   a mica particle;
   a first silicate coating at least part of the mica particle; and
   a functional substance carried by the first silicate.

2. The silicate-coated body according to claim 1, wherein at least a portion of the first silicate is joined to the mica particle via silica and/or a silica modified product.

3. The silicate-coated body according to claim 1 or 2, wherein a median particle size of the mica particle is from 0.1 μm to 10 mm.

4. The silicate-coated body according to any one of claims 1 to 3, further comprising:

an intermediate layer coating the mica particle;
wherein at least a portion of the first silicate coats the mica particle via the intermediate layer.

5. The silicate-coated body according to claim 4, wherein the intermediate layer is a coating that exhibits an interference color.

6. The silicate-coated body according to claim 4 or 5, wherein the intermediate layer comprises a metal oxide having a refractive index of 2 or greater.

7. The silicate-coated body according to any one of claims 4 to 6, wherein the intermediate layer comprises titanium dioxide, iron oxide, or a combination thereof.

8. A silicate-coated body comprising:

a substrate;
silica and/or a silica modified product present on the substrate;
a first silicate coating at least part of the substrate via the silica and/or silica modified product; and
a functional substance carried by the first silicate.

9. The silicate-coated body according to claim 8, wherein the substrate comprises particles of a second silicate.

10. The silicate-coated body according to claim 8 or 9, wherein the substrate is flaky and/or platy mica powder.

11. The silicate-coated body according to any one of claims 8 to 10, wherein the first silicate is integral with the silica and/or the silica modified product.

12. The silicate-coated body according to any one of claims 8 to 11, further comprising:
an intermediate layer coating the substrate, wherein:

the silica and/or silica modified product adheres to at least part of a surface of the intermediate layer; and
at least a portion of the first silicate coats the substrate via the intermediate layer and/or the silica and/or silica modified product.

13. The silicate-coated body according to claim 12, wherein the intermediate layer is a coating that exhibits an interference color.

14. The silicate-coated body according to claim 12 or 13, wherein the intermediate layer comprises a metal oxide having a refractive index of 2 or greater.

15. The silicate-coated body according to any one of claims 12 to 14, wherein the intermediate layer comprises titanium dioxide, iron oxide, or a combination thereof.

16. The silicate-coated body according to any one of claims 1 to 15, wherein the first silicate comprises a smectite-group silicate.

17. The silicate-coated body according to claim 16, wherein the smectite-group silicate comprises hectorite.

18. The silicate-coated body according to any one of claims 1 to 17, wherein the functional substance is ionic.

19. The silicate-coated body according to any one of claims 1 to 18, wherein the functional substance comprises at least one selected from the group consisting of antibacterial substances, bactericidal substances, sterilizing substances, and disinfecting substances.

20. The silicate-coated body according to any one of claims 1 to 19, wherein the functional substance is at least one selected from the group consisting of metal ions, ionic metal complexes, and cationic surfactants.

21. The silicate-coated body according to any one of claims 1 to 20, wherein the functional substance is at least one selected from the group consisting of a silver ion, a zinc ion, a copper ion, a diamminesilver ion, a benzalkonium ion, a benzethonium ion, a tetraethylammonium ion, and a didecyldimethylammonium ion.

22. The silicate-coated body according to any one of claims 1 to 21, further comprising:

    a multivalent cation;
    wherein the functional substance comprises an anionic functional substance and/or an amphoteric functional substance.

23. The silicate-coated body according to any one of claims 1 to 22, further comprising:
    an ionic organic colorant adhering to the first silicate.

24. The silicate-coated body according to claim 23, further comprising:

    a multivalent cation;
    wherein the ionic organic colorant comprises an anionic organic colorant and/or an amphoteric organic colorant.

25. The silicate-coated body according to claim 24, wherein the multivalent cation is at least one selected from the group consisting of a magnesium ion, a calcium ion, an aluminum ion, and a barium ion.

26. The silicate-coated body according to any one of claims 23 to 25, wherein the ionic organic colorant includes at least one selected from the group consisting of amaranth, new coccine, phloxine B, rose bengal, acid red, tartrazine, sunset yellow, fast green, brilliant blue, indigo carmine, lithol rubine B, lithol rubine BCA, methylene blue, rhodamine B, and erythrosine B.

[FIG. 1]

[FIG. 2]

[FIG. 3]

[FIG. 4]

[FIG. 5]

[FIG. 6]

```
┌─────────────────────────────┐
│      First Mixing Step      │ ⌇ S11
└─────────────────────────────┘
              │
              ▼
┌─────────────────────────────┐
│        Heating Step         │ ⌇ S12
└─────────────────────────────┘
              │
              ▼
┌─────────────────────────────┐
│        Cooling Step         │ ⌇ S13
└─────────────────────────────┘
              │
              ▼
┌─────────────────────────────┐
│    First Separating Step    │ ⌇ S14
└─────────────────────────────┘
              │
              ▼
┌─────────────────────────────┐
│      First Drying Step      │ ⌇ S15
└─────────────────────────────┘
              │
              ▼
┌─────────────────────────────┐
│     Second Mixing Step      │ ⌇ S16
└─────────────────────────────┘
              │
              ▼
┌─────────────────────────────┐
│   Second Separating Step    │ ⌇ S17
└─────────────────────────────┘
              │
              ▼
┌─────────────────────────────┐
│     Second Drying Step      │ ⌇ S18
└─────────────────────────────┘
```

[FIG. 7]

[FIG. 8]

[FIG. 9]

```
┌─────────────────────────────┐
│     Third Mixing Step       │ ～ S21
└─────────────────────────────┘
              │
              ▼
┌─────────────────────────────┐
│   Third Separating Step     │ ～ S22
└─────────────────────────────┘
              │
              ▼
┌─────────────────────────────┐
│     Third Drying Step       │ ～ S23
└─────────────────────────────┘
```

[FIG. 1 0]

[FIG. 1 1]

[FIG. 1 2]

| Intermediate Layer Forming Step | S31 |
| First Mixing Step | S32 |
| Heating Step | S33 |
| Cooling Step | S34 |

[FIG. 1 3]

[FIG. 1 4]

[FIG. 1 5]

[FIG. 1 6]

[FIG. 1 7]

[FIG. 1 8]

[FIG. 1 9]

[FIG. 2 0]

[FIG. 2 1]

[FIG. 2 2]

[FIG. 2 3]

5.0kV 8.1mm x3.50k SE(U)          10.0um

[FIG. 2 4]

5.0kV 8.1mm x8.00k SE(U)　　　5.00um

[FIG. 2 5]

5.0kV 8.1mm x60.0k SE(U)    500nm

[FIG. 2 6]

2.0kV 7.8mm x4.50k SE(U)                    10.0um

[FIG. 2 7]

2.0kV 7.8mm x20.0k SE(U)　　　　　　2.00um

[FIG. 2 8]

[FIG. 2 9]

2.0kV 7.9mm x3.00k SE(U)　　　10.0um

[FIG. 3 0]

2.0kV 8.0mm x20.0k SE(U)　　　2.00um

[FIG. 3 1]

2.0kV 8.0mm x30.0k SE(U)　　　　　1.00um

[FIG. 3 2]

2.0kV 7.6mm x2.20k SE(U)　　　　　20.0um

[FIG. 3 3]

2.0kV 7.7mm x22.0k SE(U)　　　　2.00um

[FIG. 3 4]

2.0kV 7.7mm x60.0k SE(U)　500nm

[FIG. 3 5]

2.0kV 7.9mm x8.00k SE(U)    5.00um

[FIG. 3 6]

2.0kV 7.9mm x18.0k SE(U)          3.00um

[FIG. 3 7]

2.0kV 7.9mm x18.0k SE(U)     3.00um

[FIG. 3 8]

JEOL      SE   SEM   LEI   1.0kV   X2,500   WD 8.0mm   10 $\mu$ m

[FIG. 3 9]

[FIG. 4 0]

[FIG. 4 1]

[FIG. 4 2]

[FIG. 4 3]

0hr　　　　　　　3hr　　　　　　　24hr, Separated Powder

[FIG. 4 4]

10.0μm

[FIG. 4 5 ]

[FIG. 4 6 ]

[FIG. 4 7]

[FIG. 4 8]

[FIG. 4 9]

[FIG. 5 0]

[FIG. 5 1]

[FIG. 5 2]

[FIG. 5 3]

[FIG. 5 4]

[FIG. 5 5]

[FIG. 5 6]

[FIG. 5 7]

[FIG. 5 8]

[FIG. 5 9]

[FIG. 6 0]

## INTERNATIONAL SEARCH REPORT

| International application No. |
|---|
| PCT/JP2019/032552 |

### A. CLASSIFICATION OF SUBJECT MATTER
Int.Cl. see extra sheet

According to International Patent Classification (IPC) or to both national classification and IPC

### B. FIELDS SEARCHED

Minimum documentation searched (classification system followed by classification symbols)

Int.Cl. C01B33/36, A61K9/14, A61K9/20, A61K9/26, A61K9/28, A61K31/14, A61K33/00, A61K33/30, A61K33/34, A61K33/38, A61K45/00, A61K47/02, A61K47/04, A61K47/20, A61K47/22, A61P31/00, A61P31/04, C09C1/00, C09C3/06, C09C3/08

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2014/104347 A1 (KAO CORP.) 03 July 2014, claims 1–7 & CN 104884036 A & TW 201429496 A | 1–26 |
| A | JP 2004-331878 A (SHISEIDO CO., LTD.) 25 November 2004, claims 1–14 & US 2006/0110344 A1, claims 1–39 & WO 2004/063286 A1 & EP 1589077 A1 & KR 10-2005-0094835 A | 1–26 |

☒ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13 September 2019 (13.09.2019) | 24 September 2019 (24.09.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/032552 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2008-156439 A (MERCK PATENT GMBH) 10 July 2008, claims 1-13 & US 2008/0153922 A1, claims 1-13 & EP 1935946 A2 & KR 10-2008-0059061 A & CN 101294008 A | 1-26 |
| P, A | WO 2018/150600 A1 (TOPY INDUSTRIES, LTD.) 23 August 2018, entire text (Family: none) | 1-26 |

Form PCT/ISA/210 (continuation of second sheet) (January 2015)

**INTERNATIONAL SEARCH REPORT**

International application No.

PCT/JP2019/032552

```
CLASSIFICATION OF SUBJECT MATTER
C01B33/36(2006.01)i, A61K9/14(2006.01)i, A61K9/20(2006.01)i,
A61K9/26(2006.01)i, A61K9/28(2006.01)i, A61K31/14(2006.01)i,
A61K33/00(2006.01)i, A61K33/30(2006.01)i, A61K33/34(2006.01)i,
A61K33/38(2006.01)i, A61K45/00(2006.01)i, A61K47/02(2006.01)i,
A61K47/04(2006.01)i, A61K47/20(2006.01)i, A61K47/22(2006.01)i,
A61P31/00(2006.01)i, A61P31/04(2006.01)i, C09C1/00(2006.01)i,
C09C3/06(2006.01)i, C09C3/08(2006.01)i
```

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018155738 A
- JP H07505112 A
- JP 2014024711 A
- JP H03193707 A

**Non-patent literature cited in the description**

- **TOMOHIKO OKADA et al.** Swellable Microsphere of a Layered Silicate Produced by Using Monodispersed Silica Particles. *J. Phys. Chem. C,* 2012, vol. 116, 21864-21869
- Determination of Antibacterial Activity and Efficacy of Textile Products. *JIS L 1902,* 2015